# EUROPEAN PATENT SPECIFICATION

(11) **EP 0 103 551 B1**
(45) Date of publication and mention of the grant of the patent: **26.02.1986**
(21) Application number: 83850170.8
(22) Date of filing: 20.06.1983
(51) Int. Cl.: C07D 473/18, C07D 473/00, C07D 473/02, C07D 473/30, C07D 473/32, A61K 31/52

(54) **Novel derivatives of guanine**
Guanin-Derivate
Dérivés de guanine

(30) Priority: 21.06.1982 SE 8203855
(43) Date of publication of application: 21.03.1984
(73) Proprietor: Astra Läkemedel Aktiebolag, 151 85 Södertälje (SE)
(72) Inventor: Eklind, Karin Ingeborg, S-151 33 Södertälje (SE); Gotthammar, Kristina Birgitta, S-132 00 Saltsjö-Bo (SE); Hagberg, Curt-Erik, S-194 51 Upplands Väsby (SE); Johansson, Karl Nils-Gunnar, S-150 23 Enhörna (SE); Kovacs, Zsuzsanna Maria Ilona, S-151 00 Järna (SE); Norèn, Jan-Olof, S-140 32 Grödinge (SE); Stening, Göran Bertil, S-151 50 Södertälje (SE)
(74) Representative: Wurm, Bengt Runio

## Description

### Field of the Invention

The present invention relates to novel derivatives of guanine, methods for their preparation, novel pharmaceutical compositions and use of said compounds in the treatment of virus infections, such as herpes virus infections, which can cause various diseases in an animal or human host, including both common infections and neoplastic diseases, i.e. cancer.

### Background of the Invention

The effects of viruses on bodily functions is the end result of changes occurring at the cellular and subcellular levels. The pathogenic changes at the cellular level are different for different combinations of viruses and host cells. While some viruses cause a general destruction (killing) of certain cells, others may transform cells to a neoplastic state.

Important common viral infections are herpes dermatitis (including herpes labialis), herpes keratitis, herpes genitalis, herpes zoster, herpes encephalitis, infections mononucleosis and cytomegalovirus infections all of which are caused by viruses belonging to the herpes-virus group. Other important viral diseases are influenza A and B which are caused by influenza A and B virus respectively. Another important common viral disease is viral hepatitis and especially hepatitis B virus infections are widely spread. Effective and selective antiviral agents are needed for the treatment of these diseases as well as for other diseases caused by viruses.

Several differentviruses of both DNA and RNA type have been shown to cause tumors in animals. The effect of cancerogenic chemicals can on animals result in activation of latent tumor viruses. It is possible that tumor viruses are involved in human tumors. The most likely human cases known today are leucemias, sarcomas, breast carcinomas, Burkitt lymphomas, nasopharyngeal carcinomas and cervical cancers where RNA tumor viruses and herpes viruses are indicated. This makes the search for selective inhibitors oftumorogenic viruses and their functions an important undertaking in the efforts to treat cancer.

### Prior Art

The compound 9-(4-hydroxybutyl)-guanine is disclosed in Chem. Pharm. Bull. 17 (1969) 1268-1270 and in Agr. Biol. Chem., 37 (1973) 2037-2043. However, no antiviral or other pharmacological activity has been disclosed for said compound.

US 4 199 574 discloses a broad class of substituted purines of the formula 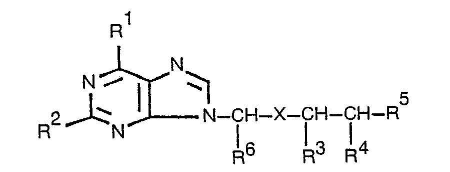 wherein X is oxygen or sulphur; R' is hydrogen, halogen, hydroxy, alkoxy, azide, thio, alkylthio, amino, alkylamino, or dialkylamino; R³ is hydrogen, halogen, alkylthio, acylamino, amino or azide; R³ is hydrogen, straight or branch chain or cyclic alkyl, hydroxyalkyl, benzyloxyalkyl, or phenyl; R⁴ is hydrogen, hydroxy or alkyl; R⁵ is hydrogen, hydroxy, amino, alkyl, hydroxyalkyl, benzyloxy, benzoyloxy, benzoyloxymethyl, sulphamoyloxy, phosphate carboxypropionyloxy, straight chain or cyclic acyloxy having from 1 to 8 carbon atoms e.g., acetoxy or substituted carbamoyl group of formula NHCO-Z wherein Z is alkyl, aryl or aralkyl optionally substituted by one or more of sulphonyl, amino, carbamoyl or halogen; R⁶ is hydrogen or alkyl, provided that when X is oxygen and'R², R³, R⁴ and R⁶ are hydrogen, R' is not amino or methylamino when R⁵ is hydrogen or hydroxy. These compounds are asserted to possess antiviral activity against various classes of DNA and RNA viruses. 9-(2-hydroxyethoxymethyl)guanine and 2-amino-9-(2-hydroxyethoxymethyl)adenine are mentioned as examples of especially active compounds.

### Disclosure of Invention

The present invention relates to the novel antiviral compound of the formula 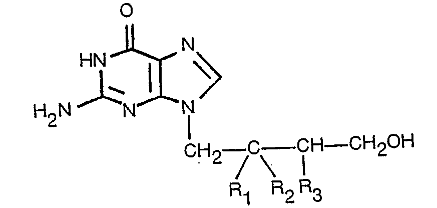 wherein R, is hydrogen, R₂ is hydrogen, fluoro, methoxy or methylthio and R₃ is hydrogen, hydroxy or mercapto; with the provisos that when R₃ is hydrogen then R₂ is methoxy or methylthio and that when R₃ is hydroxy then R₂ is fluoro, methoxy or methylthio; and with the further proviso that R, can also be methoxy or fluoro when R₂ is methoxy or fluoro; and physiologically acceptable salts or optical isomers thereof.

It has been found that such compounds exert-an antiviral effect and inhibit certain viral functions including tumorogenic functions and the multiplication of viruses.

The invention thus provides a compound, and physiologically acceptable salts thereof, which compounds are useful in therapeutic and/or prophylactic treatment of viral diseases and which may be useful in therapeutic and/or prophylactic treatment of cancer caused by viruses.

An effective selective antiviral agent with acceptable side effects should have a selective inhibiting effect on a specific viral function of the virus to be combated. It is, therefore, one object of the present invention to provide compounds to be used in a novel method for combating virus infections, which compounds exert a selective inhibiting effect on viral functions but which exert only a negligible inhibiting effect on functions of the host cells.

The invention also relates to novel pharmaceutical compositions containing the antiviral agents.

Although the present invention relates broadly to compounds to be used in a novel method for combating virus infections in animals and man, it will be particularly useful in the treatment of herpesvirus infections.

An especially important area of use for the compounds of the present invention is in the treatment of herpesvirus infections. Among the herpesviruses may be mentioned Herpes simplex type 1 and 2, varicella (Herpes zoster), virus causing infectious mononucleosis (i.e. Epstein-Barr virus) and cytomegalovirus. Important diseases caused by herpesviruses are herpes dermatitis (including herpes labialis), herpes genitalis, herpes keratitis, herpes encephalitis and herpes zoster.

Another possible area of use for the compounds of the present invention is in the treatment of cancer and tumors, particularly those caused by viruses. This effect may be obtained in different ways, i.e. by inhibiting the transformation of virus-infected cells to a neoplastic state, by inhibiting the spread of viruses from transformed cells to other normal cells and by arresting the growth of virustransformed cells.

A further area of use for the compounds of the present invention is in the inhibition of transformed cells due to the presence in these cells of specific herpesvirus enzymes like thymidine kinase.

Possible areas of use for the compounds of the present invention with respect to cancer chemotherapy are treatment of leucemias, lymphomas including Burkitt lymphomas and Hodgkin's disease, sarcomas, breast carcinoma, nasopharyngeal carcinomas and cervical cancers in which viruses are indicated. Other possible areas of use for the compounds of the present invention with respect to cancer chemotherapy are treatment of multiple myeloma and cancer of the lungs (and bronchus), the stomach, the liver, the colon, the bladder, the lips, the bones, the kidneys, the ovary, the prostate, the pancreas, the skin (melanoma), the rectum, the salivary glands, the mouth, the esophagus, the testis, the brain (and cranial meninges), the thyroid gland, the gallbladder (and ducts), the nose, the larynx, connective tissues, the penis, the vulvas, the vagina, the corpus uteri and the tongue.

The invention furthermore provides
A. The compound of the formula I for use in the treatment of diseases caused by viruses in animals including man, comprising administering to an animal so infected a therapeutically effective amount of a said compound or a physiologically acceptable salt thereof.
B. The compound of the formula I for use in inhibiting the multiplication of virus, in particular herpesviruses, in animals including man, by administering to an animal in need of such treatment a said compound of a physiologically acceptable salt thereof in an amount sufficient for inhibiting said multiplication.
C. The compound of the formula for use in the treatment of virus-induced neoplastic diseases in animals including man, by inhibiting the growth of cells expressing viral functions, characterized by administering to an animal so infected a therapeutically effective amount of a said compound or a physiologically acceptable salt thereof.
D. The compound of the formula I for use in inhibiting the growth of virus-transformed cells in animals including man, characterized by administering to an animal in need of such treatment a said compound of physiologically acceptable salt thereof in an amount sufficient for inhibiting said growth.
E. The compound of the formula I for use in the treatment of virus-induced neoplastic diseases in animals including man, by inhibiting the multiplication of tumor viruses, characterized by administering to an animal in need of such treatment a said compound or a physiologically acceptable salt thereof in an amount sufficient for inhibiting said multiplication.
F. The compound of the formula I for use in the treatment of neoplastic diseases in animals including man, characterized by administering to an animal a therapeutically effective amount of a said compound or a physiologically acceptable salt thereof.

As stated previously the compound of the present invention has the formula 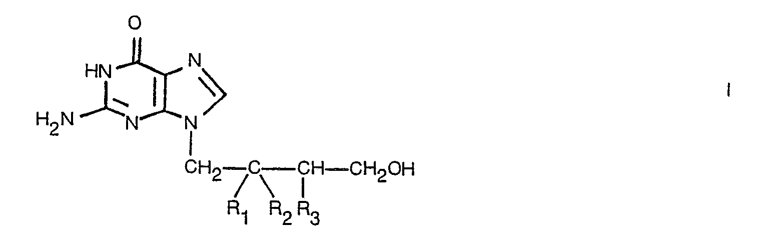 wherein R, is hydrogen, R₂ is hydrogen, fluoro, methoxy or methylthio and R₃ is hydrogen, hydroxy or mercapto; with the provisos that when R₃ is hydrogen then R₂ is methoxy or methylthio and that when R₃ is hydroxy then R₂ is fluoro, methoxy or methylthio; and with the further proviso that R, can also be methoxy or fluoro when R₂ is methoxy or fluoro; including physiologically acceptable salts and optical isomers thereof.

Preferred subgroups of said compounds in accordance with the invention are those wherein:
(I) R₃ is selected from hydrogen and hydroxy, R₁ is selected from hydrogen, fluoro and methoxy and R₂ is selected from fluoro, methoxy and methylthio, with the provisos that when R, is hydrogen and R₂ is fluoro then R₃ is hydroxy and that when R₁ is fluoro or methoxy then R₂ is also fluoro or methoxy; or
(II) R₃ is hydrogen, R₁ is selected from hydrogen, fluoro and methoxy and R₂ is selected from fluoro, methoxy and methylthio, with the proviso that when R, is fluoro or methoxy, then R₂ is also fluoro or methoxy; or
(III) R, and R₂ are the same or different and are selected from hydrogen and fluoro, and R₃ is selected from hydrogen and hydroxy, with the proviso that when R₁ is hydrogen and R₂ is fluoro, then R₃ is hydroxy; or
(IV) R₁ is selected from hydrogen and fluoro, R₂ is selected from hydrogen, fluoro, methoxy and methylthio and R₃ is selected from hydrogen and hydroxy, with the provisos that when R₁ is hydrogen and R₂ is fluoro, then R₃ is hydroxy and with the further proviso that when R₁ is fluoro, then R₂ is fluoro or methoxy; or
(V) R₁ is selected from hydrogen, fluoro and methoxy, R₂ is selected from hydrogen, fluoro, methoxy and methylthio and R₃ is selected from hydrogen and hydroxy, with the provisos that when R₁ is hydrogen and R₂ is fluoro, then R₃ is hydroxy and that when R₁ is fluoro or methoxy, then R₂ is also fluoro or methoxy; or
(VI) R₁ is selected from hydrogen and fluoro, R₂ is selected from hydrogen, fluoro, methoxy and methylthio and R₃ is selected from hydrogen and mercapto, with the provisos that when R₁ is hydrogen and R₂ is hydrogen or fluoro, then R₃ is mercapto; and that when R₁ is fluoro, then R₂ is fluoro or methoxy; or
(VII) R₃ is selected from hydrogen and mercapto, R₁ is selected from hydrogen, fluoro and methoxy and R₂ is selected from hydrogen, fluoro, methoxy and methylthio, with the provisos that when R₁ is hydrogen and R₂ is hydrogen or fluoro, then R₃ is mercapto and that when R₁ is fluoro or methoxy, then R₂ is also fluoro or methoxy; or
(VIII) R₁ and R₂ are the same or different and are selected from hydrogen and fluoro and R₃ is selected from hydrogen, hydroxy and mercapto with the provisos that when R₁ and R₂ are both hydrogen, then R₃ is mercapto and that when R₁ is hydrogen and R₂ is fluoro, then R₃ is selected from hydroxy or mercapto; or
(IX) R₁ and R₂ are the same or different and are selected from hydrogen, fluoro and methoxy and R₃ is selected from hydrogen, hydroxy and mercapto, with the proviso that when R₁ and R₂ are both hydrogen, then R₃ is mercapto and that when R₁ is hydrogen and R₂ is fluoro, then R₃ is selected from hydroxy or mercapto; or
(X) R₁ is hydrogen, R₂ is selected from hydrogen, methoxy and methylthio and R₃ is selected from hydrogen, hydroxy and mercapto, with the proviso that when R₁ and R₂ are both hydrogen, then R₃ is mercapto; or
(XI) R₁ is hydrogen, R₂ is selected from hydrogen, fluoro, methoxy and methylthio and R₃ is selected from hydrogen, hydroxy and mercapto, with the provisos that when R₂ is fluoro, then R₃ is selected from hydroxy and mercapto and that when R₁ and R₂ are both hydrogen, then R₃ is mercapto; or
(XII) R₁ is hydrogen, R₂ is selected from hydrogen and fluoro and R₃ is selected from hydroxy and mercapto, with the provisos that when R₂ is hydrogen, then R₃ is mercapto and that when R₂ is fluoro, then R₃ is selected from hydroxy and mercapto; or
(XIII) R₁ is hydrogen, R₂ is selected from hydrogen, fluoro, methoxy and methylthio and R₃ is selected from hydrogen, hydroxy and mercapto, with the provisos that when R₂ is hydrogen, then R₃ is mercapto and that when R₂ is fluoro then R₃ is selected from hydroxy and mercapto.

The provisos in the definition for the groups R₁, R₂ and R₃ above mean that the following specific compounds, including salts and optical isomers thereof, constitute part of the present invention:

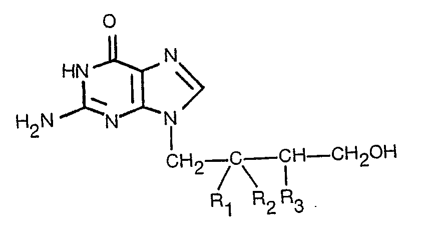

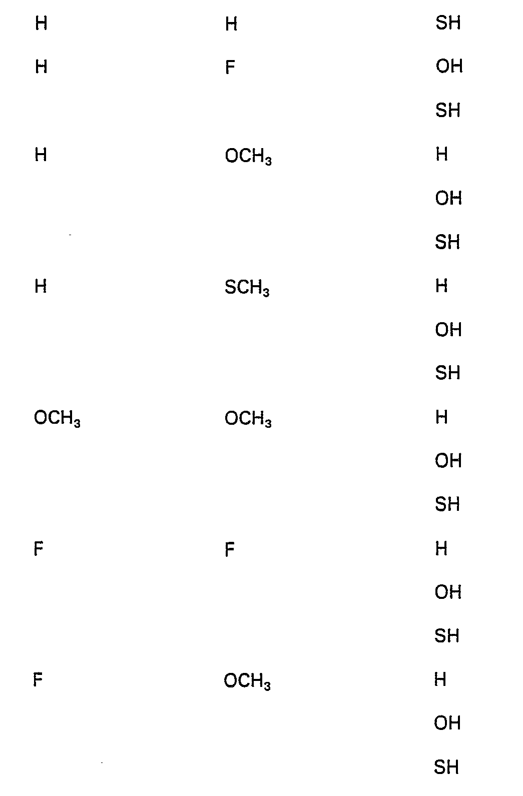

The compounds of the formula I contain one or two asymmetric centers. Accordingly, they exist in two or four optical forms, respectively, and all such forms as well as the diastereomeric isomers constitute a further aspect of the invention.

### Methods of Preparation

The compounds of the invention may be obtained by one of the following methods A-D constituting a further aspect of the invention.

A. Condensing an acyclic side chain, where the functional groups may optionally be protected, to the N-9 position of a guanine derivative, followed by removal of the protecting groups, through one or more chemical reactions. 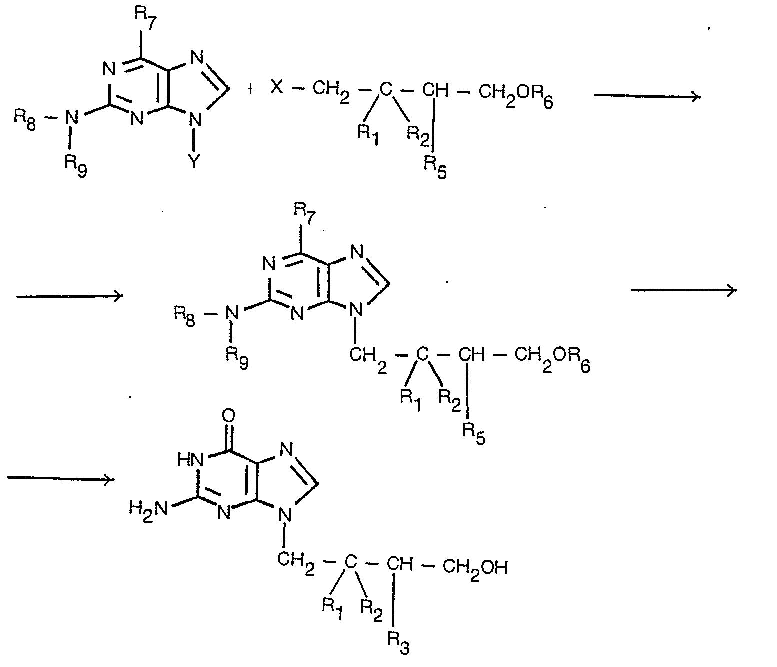 wherein R₁ and R₂ has the meaning given above, X is chlorine, bromine, iodine or a group OSO₂R₁₀ where R₁₀ is alkyl containing 1―8 carbon atoms, fluorinated alkyl containing 1―8 carbon atoms such as trifluoromethyl, alkylaryl such as benzyl or aryl, Y is hydrogen or a quarternary ammonium ion such as for example tetrabutylammonium.

R₅ is R₃ as defined above, or OR₆ or SR₆ where R₈ is hydrogen or a hydroxyl or a mercapto protecting group of which a great variety are known to those skilled in the art and are described for example in "Protective Groups in Organic Chemistry" (T. W. Greene, Wiley 1981), "Methoden der Organischen Chemie" (Houben-Weyl)" VI/Ib, or in "Comprehensive Organic Chemistry" (D. H. R. Barton and W. D. Ollis eds., 1979) Vol. 1, p. 623―629.

Just some examples of R₆ are acyl groups such as acetyl or benzoyl, alkoxy carbonyl or aryloxycarbonyl groups, silyl groups such as for example tert.-butyl dimethylsilyl, alkylaryl such as benzyl and triarylmethyl, or SO₂R₁₀ where R₁₀ is as defined above.

R₅ and OR₆ may together form an epoxide when R₃ is OH and R₅ and OR₆ may additionally form a cyclic derivative such as for example a carbonate ester or carbonate thioester or the corresponding orthoacid cyclic derivatives or cyclic acetal type compounds.

R₇ is hydroxyl, chlorine, bromine, iodine, thiol, thioether, SO₂R₁₀ where R₁₀ is as defined above; or an oxygen derivative OR₁₂ where R₁₂ is alkyl, alkylaryl such as benzyl, substituted silyl, phosphoryl diester, phosphinothioyl or SO₂R₁₀ where R₁₀ is as defined above. R₈ and R₉ are the same or different and are R₁₁ where R₁₁ is hydrogen or an amine protecting group known to those skilled in the art and described for example in "Protective Groups in Organic Chemistry" (T. W. Greene, Wiley 1981), "Methoden der Organischen Chemie (Houben-Weyl)" XI/1 p. 1005 cont., or in "Comprehensive Organic Chemistry" (D. H. R. Barton and W. D. Ollis eds., 1979) Vol. 2, p. 49―52. Some examples of R₁₁ are acyl groups, alkoxycarbonyl or aryloxycarbonyl groups, or silyl groups.

The condensation is preferably conducted in an organic solvent such as for example dimethylformamide, ethanol, acetonitrile or dichloromethane, at a temperature of between 0°C and 100°C for 1 hour to 3 days in the presence of a base (when Y is H) such as for example potassium carbonate.

After condensation, the compounds are, if necessary, hydrolyzed at 0―100°C for 1―24 hours with acid or base such as for example acetic acid, hydrochloric acid (1-35%) in water, sodium hydroxide (1-20%) in water, ammonia (1-25%) in water or methanol, or hydrogenated with hydrogen gas in an organic solvent such as for example ethanol or dimethylformamide over a metal tatalyst for 1-24 hours at a pressure of 0.1-5 MPa.

B 1. By substitution reactions on a guanine N-9 derivatized 4-carbon side-chain.

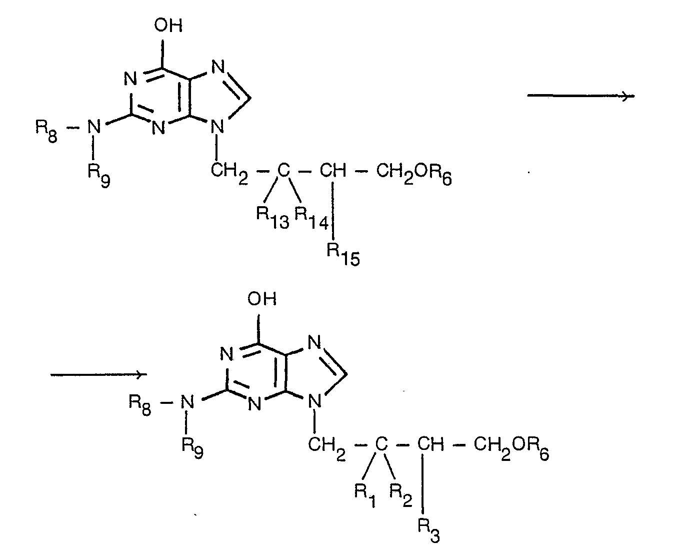

R₆, R₈ and R₉ are as defined above. R₁₃ is R₁, iodine or OSO₂R₁₀, R₁₄ is R₂, iodine or OSO₂R₁₀, R₁₅ is R₃, iodine or OSO₂R₁₀ where R₁, R₂, R₃ and R₁₀ are as defined above. The substitution reactions are performed in an organic solvent such as dimethylformamide, ethanol, acetonitrile or dichloromethane at a temperature of between 0°C and 100°C for 1-24 hours and the substituting reagent will be hydrogen fluoride, methanol, methane thiol, water, ammonia or hydrogen sulfide or their respective ions or ion pairs.

When R₆, R₈ and R₉ are not hydrogen then in a following reaction step R₆, R₈ and R₉ are removed according to method A.

B 2. By reduction of an ester group to an alcohol.

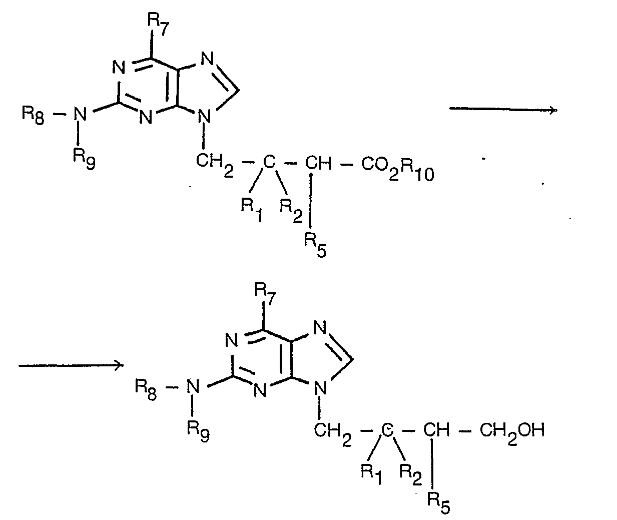

R₁, R₂, R₅, R₇, R₈, R₉ and R₁₀ are as defined above. The reduction may be performed by hydrogen gas or hydrogen generated in situ, with a metal as catalyst or with a hydride reducing agent in an organic solvent. When R₇ is not hydroxy, R₈ and R₉ are not hydrogen and R₅ is not R₃, then the protecting groups are removed in a following reaction step according to method A.

C 1. Pyrimidine ring closure to the guanine base of a substituted imidazole derivative 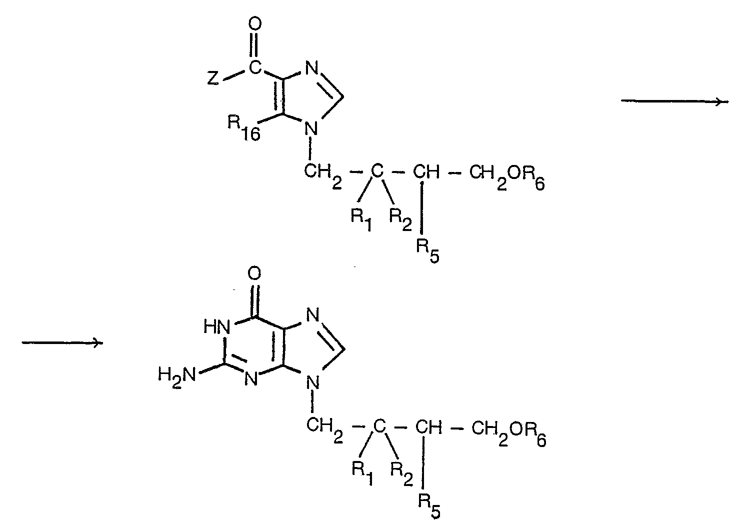 wherein R₁, R₂, R₅ and R₆ are as defined above, Z is NH₂ or alkoxy i.e. COZ is an amide or ester group and R₁₆ is NH₂, or guanidine. The ring closure may be performed by known methods, the principles of which are given for example in "Comprehensive Organic Chemistry" p. 505-508 (1979, vol. 4, D. H. R. Barton and W. D. Ollis eds.).

The ring closure is performed in an organic solvent at a temperature from 50° to 250°C with or without the addition of a reagent such as for example guanidine. When R₆ is not H and R₅ is not R₃, then the side chain protecting groups are removed in a following reaction step according to method A.

C 2. Imidazole ring closure, to the guanine base, of a substituted pyrimidine derivative. 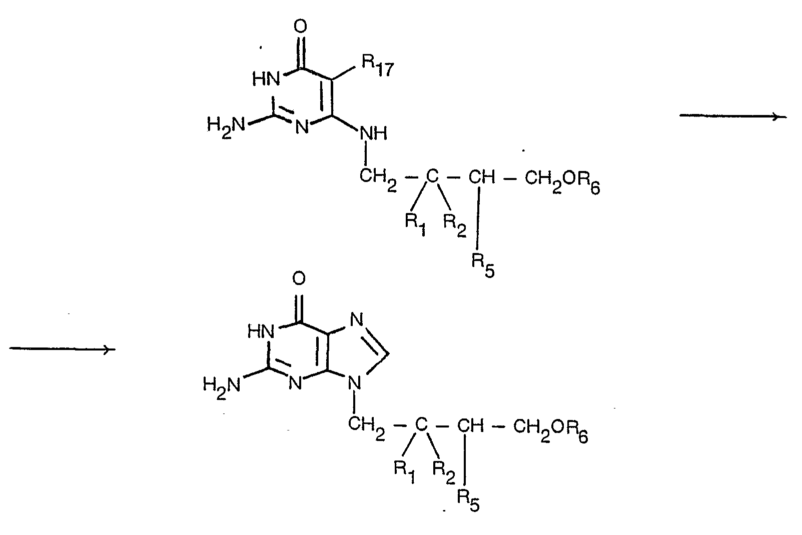 wherein R₁, R₂, R₅ and R₈ are as defined above and R₁₇ is nitroso, nitro, amino, or an amino derivative such as formic amide (-NH-CHO) or amino ortho ester 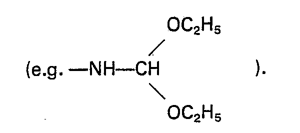 The ring closure may be performed by known methods, the principles of which are given for example in "Comprehensive Organic Chemistry" p. 499-504 (1979, Vol. 4, D. H. R. Barton and W. D. Ollis eds.).

The ring closure may be performed in an organic solvent such as for example formic acid, formamide, orthoformate ester at a temperature from 50 to 250°C for hour to 10 hours. When R₁₇ is nitroso or nitro, these groups first have to be reduced to amino groups by known methods. When R₆ is not H and Rₛ is not R₃, then the side chain protecting groups are removed in a following reaction step according to method A.

D. Substitution in the pyrimidine ring of a purine to form a guanine ring

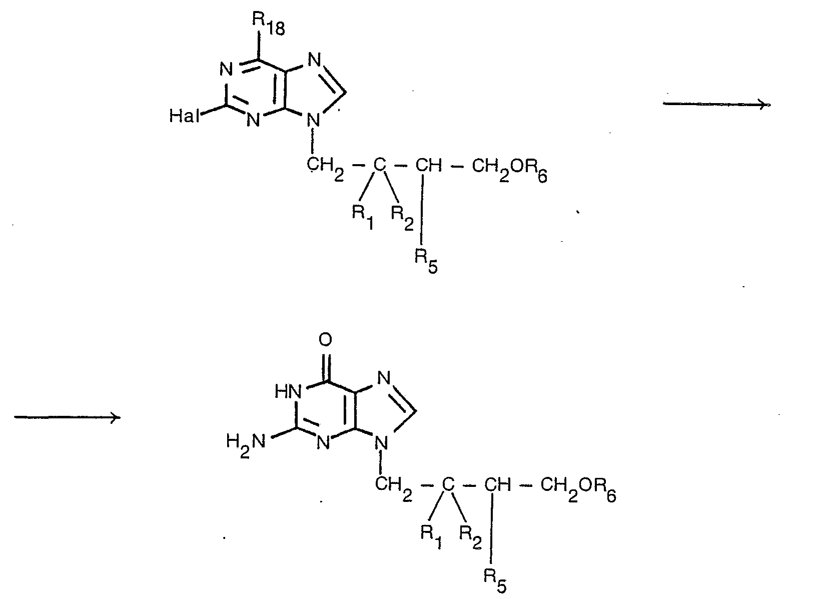

Rᵢ, R₂, R₅, R₆ and Hal have the meaning given above and R₁₈ is hydroxyl or amino. The halogen atoms are substituted by ammonia in an organic solvent such as methanol, from normal to higher pressure at room temperature to 100°C for 1 to 25 hours or by an azide ion followed by hydrogenation by known methods. When R₁₈ is amino the amino group can be replaced by a hydroxyl function by selective diazotization with nitrite in a solvent such as acetic acid at a temperature from 0°C to 50°C for 1-24 hours, or enzymatically with adenosinedeaminase in water at a pH from 6 to 9 from 1 to 48 hours. When R₆ is not hydrogen and Rₛ is not R₃, then the side chain protecting groups are removed in a following reaction step according to method A.

The described methods A-D may be used to give mixtures of diastereomers and optical isomers, or in appropriate cases a single diastereomer or a single optical isomer. Additionally a single optical isomer may be obtained from the optical mixtures by methods known per se.

The starting materials in the above methods A-D are either known compounds or can be prepared by methods known to those skilled in the art.

### Salts

Physiologically acceptable salts of compounds of the invention are prepared by methods known in the art. The salts are novel compounds and comprise a further aspect of the invention. Metal salts can be prepared by reacting a metal hydroxide with a compound of the invention. Examples of metal salts which can be prepared in this way are salts containing Li, Na and K. A less soluble metal salt can be precipitated from a solution of a more soluble salt by addition of a suitable metal compound. Acid salts can be prepared by reacting a compound of the invention with an acid such as HCI, HBr, H_{Z}SO₄, or an organic sulphonic acid.

### Pharmaceutical compositions

Pharmaceutical compositions of the compounds of the invention constitute a further aspect of the invention.

In clinical practice the compounds of the invention may be administered locally or systemically. They will normally be administered topically, orally, intranasally, by injection or by inhalation in the form of a pharmaceutical composition comprising the active ingredient in the form of the original compound or optionally in the form of a pharmaceutically acceptable salt thereof, in association with a pharmaceutically acceptable carrier which may be a solid, semi-solid or liquid diluent or an ingestible capsule, and such compositions comprise a further aspect of the invention. The compound may also be used without carrier material. As examples of pharmaceutical compositions may be mentioned tablets, drops, such as nasal drops, eye drops, preparations for topical application such as ointments, jellies, creams and suspensions, aerosols for inhalation, nasal spray, liposomes etc. Usually the active substance will comprise between 0.01 and 99, or between 0.1 and 99% by weight of the composition, for example between 0.5 and 20% for compositions intended for injection and between 0.1 and 50% for compositions intended for oral administration.

The compositions are preferably in dosage unit form. Further, they are preferably provided in sterilized form.

To produce pharmaceutical compositions in the form of dosage units for oral application containing a compound of the invention the active ingredient may be mixed with a solid, pulverulent carrier, for example lactose, saccharose, sorbitol, mannitol, a starch such as potato starch, corn starch, amylopectin, laminaria powder or citrus pulp powder, a cellulose derivative or gelatine and also may include lubricants such as magnesium or calcium stearate or a Carbowax@ or other polyethylene glycol waxes and compressed to form tablets or cores for dragees. If dragées are required, the cores may be coated for example with concentrated sugar solutions which may contain gum arabic, talc and/or titanium dioxide, or alternatively with a film forming agent dissolved in easily volatile organic solvents or mixtures of organic solvents. Dyestuffs can be added to these coatings, for example, to distinguish between different contents of active substance. For the preparation of soft gelatine capsules consisting of gelatine and, for example, glycerol and a plasticizer, or similar closed capsules, the active substance may be admixed with a Carbowax@ or a suitable oil as e.g. sesame oil, olive oil, or arachis oil. Hard gelatine capsules may contain granulates of the active substance with solid, pulverulent carriers such as lactose, saccharose, sorbitol, mannitol, starches (for example potato starch, corn starch or amylopectin), cellulose derivatives or gelatine, and may also include magnesium stearate or stearic acid as lubricants.

By using several layers of the active drug, separated by slowly dissolving coatings sustained release tablets are obtained. Another way of preparing sustained release tablets is to divide the dose of the active drug into granules with coatings of different thicknesses and compress the granules into tablets together with the carrier substance. The active substance can also be incorporated in slowly dissolving tablets made for instance of fat and wax substances or evenly distributed in a tablet of an insoluble substance such as a physiologically inert plastic substance.

Liquid compositions for oral application may be in the form of elixirs, syrups or suspensions, for example solutions containing from about 0.1 % to 20% by weight of active substance, sugar and a mixture of ethanol, water, glycerol, propylene glycol and optionally aroma, saccharine and/or carboxymethylcellulose as a dispersing agent.

For parenteral application by injection compositions may comprise an aqueous solution of the active drug or a physiologically acceptable salt thereof, desirably in a concentration of 0.05-10%, and optionally also a stabilizing agent and/or buffer substances in aqueous solution. Dosage units of the solution may advantageously be enclosed in ampoules.

For topical application, especially for the treatment of herpesvirus infections on skin, genitals and in mouth and eyes the compositions are suitably in the form of a solution, ointment, gel, suspension, cream or the like. The amount of active substance may vary, for example between 0.05-20% by weight of the active substance. Such compositions for topical application may be prepared in known manner by mixing the active substance with known carrier materials such as isopropanol, glycerol, paraffin, stearyl alcohol, polyethylene glycol, etc. The pharmaceutically acceptable carrier may also include a known chemical absorption promoter. Examples of absorption promoters are e.g. dimethylacetamide (US 3,472,931), trichloroethanol or trifluoroethanol (US 3,891,757), certain alcohols and mixtures thereof (GB 1,001,949).

The dosage at which the active ingredients are administered may vary within a wide range and will depend on various factors such as for example the severity of the infection, the age of the patient, etc., and may have to be individually adjusted. As a possible range for the amount of the compounds of the invention which may be administered per day may be mentioned from about 0.1 mg to about 2000 mg or from about 1 mg to about 2000 mg, or preferably from 1 mg to about 200 mg for topical administration, from 50 mg to about 2000 mg or from 100 to about 1000 mg for oral administration and from 10 mg to about 2000 mg or from 50 to about 500 mg for injection.

In severe cases it may be necessary to increase these doses 5-fold to 10-fold. In less severe cases it may be sufficient to use up to 500 or 1000 mg.

The pharmaceutical compositions containing the active ingredients may suitably be formulated so that they provide doses within these ranges either as single dosage units or as multiple dosage units.

Thus, it has been found according to the invention that the compounds of the formula I and the physiologically acceptable salts thereof can be used to inhibit herpesvirus multiplication. The compounds of the formula I and physiologically acceptable salts thereof are useful in therapeutic and/or prophylactic treatment of virus infections.

A preferred aspect of the invention is the use of the compounds of the formula I or a physiologically acceptable salt thereof, in the treatment of herpesvirus infections.

### Working Examples

The following examples illustrate the preparation of compounds according to the invention.

### Example 1

### 9-(4-hydroxy-2,2-dimethoxybutyl)guanine

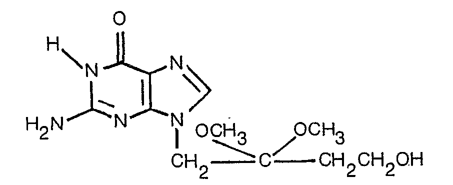

A small amount of 9-(2-oxo-4-hydroxybutyl)guanine (1.0 mg) was dissolved in dry methanol (2.5 ml) and molecular sieve 3A (0.5 g) was added. The stirred solution was treated with hydrogen chloride saturated methanol (5 drops) and kept at room temperature for 24 h. Molecular sieve 5A (0.5 g) was added and the solution was stirred for a few minutes. The reaction mixture was filtered and the filtrate was evaporated to dryness. The product was analyzed by reverse phase high performance liquid chromatography (Waters HPLC, radial pak column RP-18 8x100 mm, water-methanol 7:3, 2 ml/min). The relative retention times for the starting material and the desired product were 2.13 and 4.95 respectively.

9-(2-oxo-4-hydroxybutyl)guanine used as a starting material was prepared as follows:

### a) 3-Hydroxybutyl o-chlorobenzoate

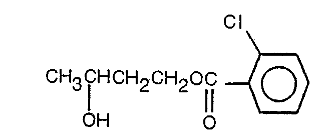

Butan 1,3-diol (1.0 g) was dissolved in pyridine (50 ml) and cooled to 0°C. Ortho-chlorobenzoylchloride (1.9 g) was added with stirring. The reaction mixture was kept at 0°C for 1 hour and then at room temperature over night. The solution was poured with stirring into ice-water (-100 ml). After stirring for 15 min the aqueous mixture was extracted with chloroform. The combined chloroform extracts were washed with ice-cold 0.25 M aqueous sulfuric acid, saturated aqueous sodium hydrogen carbonate and water. The dried solution (sodium sulfate) was concentrated to a syrup and purified by silica gel column chromatography (toluene-ethyl acetate 1:1) to yield chromatographically pure 3-hydroxybutyl o-chlorobenzoate (1.92 g) R_{F}: 0.56, TLC in the same solvent. NMR (CDCl₃): δ 1.24 (3H, d,―CH₃), 1.63―2.10 (2H, m, ―CH2―), 3.69―4.23 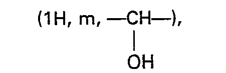 4.29―4.66 (2H, m, ―CH₂―), 7.13―7.52 (3H, m, aromatic protons), 7.63―7.92 (1H, m, aromatic proton).

### b) 3-Oxobutyl o-chlorobenzoate

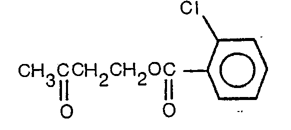

Pyridinium chlorochromate adsorbed to aluminium oxide (33 g, 32.8 mmol) (CrO₃·C₅H₅N·HCl prepared according to Yu-Shia Cheng et al., Synthesis, March 1980) was added to 3-hydroxybutyl o-chlorobenzoate (1.8 g, 7.8 mmol; prepared according to a)) in n-hexane (100 ml). After stirring at room temperature over night the solid was filtered and the filtrate evaporated to dryness to give 1.21 g of 3-oxobutyl o-chlorobenzoate (TLC toluene-ethyl acetate 1:1, R_{f} 0.74). ¹H NMR (CDCl₃): δ 2.21 (3H, s-CH₃), 2.81 (2H, triplet ―COCH₂―), 4.54 (2H, triplet ―CH₂O―), 7.0―7.45 (3H, m, aromatic protons), 7.45―7.84 (1H, m, aromatic proton).

### c) 4-Bromo-3-oxobutyl o-chlorobenzoate

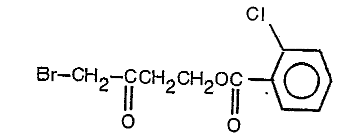

3-Oxobutyl o-chlorobenzoate (670 mg, 2.96 mmol; prepared according to b)) was dissolved in anhydrous methanol (10 ml). The solution was stirred and cooled in an icebath, and bromine (0.15 ml, 2.96 mmol) was added rapidly. The reaction mixture was kept at +5°C for 18 h. Water (7 ml) and concentrated sulfuric acid (0.6 ml) were added and the mixture was stirred at room temperature over night.

Water (10 ml) was added to the solution and the aqueous mixture was extracted with chloroform. The combined chloroform extracts were washed with saturated aqueous sodium hydrogen carbonate and water. The dried solution (sodium sulfate) was concentrated to a syrup and purified by silica gel column chromatography (toluene-ethyl acetate 16:1) to yield pure 4-bromo-3-oxobutyl o-chlorobenzoate 385 mg (TLC in the same solvent R_{f} 0.40). ¹H NMR (CDCl₃): δ 3.12 (2H, triplet COCH₂), 3.87 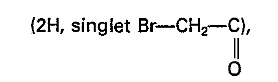 4.57 (2H,⁻ triplet ―CH₂O), 7.12―7.48 (3H, m, aromatic protons), 7.48―7.81 (1H, aromatic proton).

### d) 4-(2-Amino-6-chloropurin-9-yl)-3-oxobutyl o-chlorobenzoate

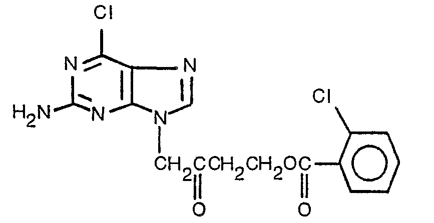

2-Amino-6-chloropurine (36.6 mg, 0.216 mmol) and anhydrous potassium carbonate (30.0 mg, 0.216 mmol) were mixed with dimethylformamide (7 ml). The mixture was cooled in an icebath and 4-bromo-3-oxobutyl o-chlorobenzoate (66 mg, 0.216 mmol; prepared according to c)) in dimethylformamide (1 ml) was added. After stirring at room temperature for 18 h, the mixture was filtered and the filtrate was evaporated to dryness. The residue was purified by preparative silica gel layer chromatography (chloroform-methanol 6:1) to yield pure 4-(2-amino-6-chloropurin-9-yl)-3-oxobutyl o-chlorobenzoate (18 mg).

TLC in the same solvent, R_{f}: 0.40.

NMR (DMSO-d₆): δ 3.15 (2H, triplet ―COCH₂―), 4.26 (2H, triplet, ―CH₂O―), 5.09 (2H, singlet ―NCH₂―CO―), 6.96 (2H, singlet, NH₂ purine residue), 7.437.63 (3H, m, aromatic protons), 7.83―7.95 (1H, m, aromatic proton), 8.06 (1H, s, H-8, purine).

### e) 9-(2-Oxo-4-hydroxybutyl)guanine

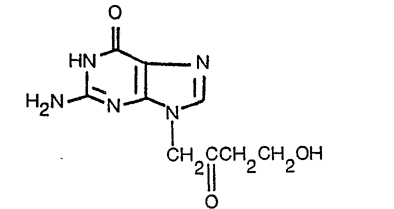

4-(2-Amino-6-chloropurin-9-yl)-3-oxobutyl o-chlorobenzoate (18 mg; prepared according to d)) was dissolved in 1 M hydrochloric acid (20 ml) and refluxed for three hours. The solution was made alkaline with diluted ammoniumhydroxide and evaporated to dryness. The residue was purified by preparative HPLC on a reverse phase column (µ Bondapack C₁₈ methanolwater 1:3), followed by chromatography on a Biogel P-2 column eluated with water. The residue, 5.4 mg, was a white solid which gave a single peak on HPLC (same solvent system as above).

NMR (DMSO-dₑ): 6 3.1 (2H, triplet, -COCH2-), 4.1 (2H, -NCH₂CO-), 4.3 (2H, triplet, -CH₂0-), 6.6 (2H, singlet, NH₂, purine residue), 7.7 (1H, singlet H-8, purine).

### Example 2

### 9-(3-Mercapto-4-hydroxybutyl)guanine

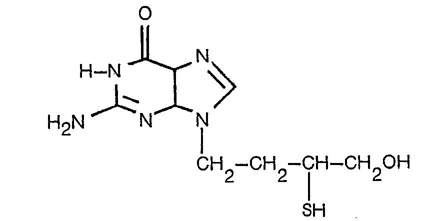

9-(3-Mercapto-4-t-butyldimethylsilyloxybutyl)guanine was dissolved in 80% acetic acid and heated on a steam bath for 1 hour. The solution was made alkaline with aqueous ammonia and concentrated. The residue was purified by preparative HPLC on a reverse phase column (µ Bondapack C₁₈ methanol-water 10:90) to give a single peak.

The starting compound, 9-(3-mercapto-4-t-butyldimethylsilyloxybutyl)guanine was prepared as follows:

### a) 4-Bromo-2-hydroxybutyric acid ethyl ester

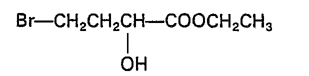

2-Hydroxybutyrolactone, GB 688.253 [CA 48 p. 3996 (1954)], (5.1 g) was dissolved in 10 ml ethanol and the solution was saturated with hydrogen bromide at 0°C. After standing at room temperature during 66 hours, the solvent was evaporated at a low pressure. The residue was mixed with ice-water and the mixture neutralized with 10% aqueous sodium carbonate. The mixture was then extracted several times with diethyl ether and the combined ether extracts washed with saturated, aqueous sodium sulphate and dried over anhydrous sodium sulphate. After evaporation of the solvent, the residue was distilled at 1,6 kPa. The fraction boiling at 109-112°C weighing 3.79 g was used in b) below.

### b) 4-(2-Amino-6-chloropurin-9-yl)-2-hydroxybutyric acid ethyl ester

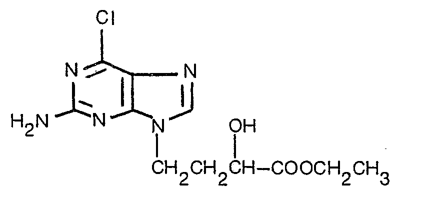

2-Amino-6-chloropurine (0.509 g, 3.00 mmole), 4-bromo-2-hydroxybutyric acid ethyl ester (0.633 g, 3.00 mmole; prepared according to a) and anhydrou's potassium carbonate (0.415 g, 3.00 mmole) were mixed with 10 ml of dimethyl formamide and the mixture stirred at room temperature during 65 hours. The mixture was then filtered and the filtrate evaporated at a pressure of 0.01 kPa. The crystalline residue was triturated with 8 ml of chloroform and the undissolved material filtered off and washed with 2 ml of chloroform. The material obtained was then triturated with 5 ml of water and the undissolved material filtered off and washed with 2 ml of water.

Recrystallization from 11 ml of ethanol gave 0.360 g product.

M.P. 163―4°C (uncorr.) UV spectrum (ethanol): λₘₐₓ (nm) 311, 248. Analyses ― Found: C 43,90; H 4.78; Cl 11.72; N 23.52; O 15.90%. Calculated for C₁₁H₁₄ClN₅O₃: C 44.08; H 4.71; Cl 11.83; N 23.37; O 16.01%.

### c) 4-(2-Amino-1,6-dihydro-6 oxopurin-9-yl)-2-hydroxybutyric acid

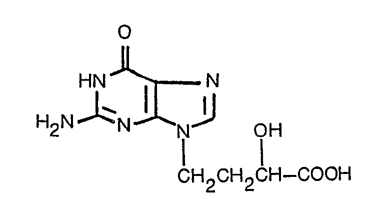

4-(2-Amino-6-chloropurin-9-yl)-2-hydroxybutyric acid ethyl ester (1.40 g, 4.67 mmole; prepared according to b) in 100 ml of 1M aqueous hydrochloric acid was refluxed during 2.5 h. The solution was then evaporated at a pressure of about 1.3 kPa. Water (25 ml) was added to the residue and the solution evaporated again. This procedure was repeated 4 times. The residue was triturated with 150 m1 of acetone and the semi-solid material filtered off to yield 1.38 g. This crude product (1.27 g) was partly dissolved in 5 ml of water, the solution was filtered and the undissolved material washed with 2.5 ml water. The pH of the filtrate was then adjusted to 6-7 with solid sodium bicarbonate. The water solution obtained was filtered and undissolved material washed with 7.5 ml water. 0.4 ml of acetic acid was then added to the filtrate. After cooling to 0°C, the precipitate was filtered off and washed with 3 ml of water. Recrystallization from 75 ml of water gave 0.68 g product. M.p. >250°C (dec.) UV spectrum (0.1M hydrochloric acid): λₘₐₓ (nm) 279, 254; UV spectrum (0.1M sodium hydroxide): λₘₐₓ (nm) 269, 256 (infl.).

Analyses - Found: C 42.63; H 4.41; N 27.74; 0 25.30%.

Calculated for C₉H₁₁N₅O₄: C 42.69; H 4.38; N 27.66; 0 25.27.

### d) 4-(2-Amino-1,6-dihydro-6-oxopurin-9-yl)-2-hydroxybutyric acid ethyl ester

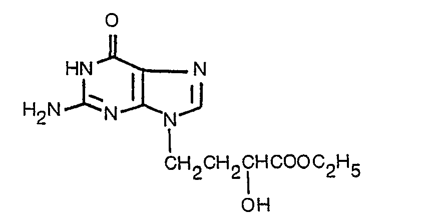

4-(2-Amino-1,6-dihydro-6-oxopurin-9-yl)-2-hydroxybutyric acid (2.00 g, 7.9 mmole; prepared according to c)) was mixed with 500 ml of ethanol The mixture was saturated with hydrogen chloride gas, first without cooling and then with cooling in ice-water. The total addition time was about 15 minutes. The mixture was then slowly warmed to room temperature and allowed to stand over night. After evaporation of the solvent, the residue was treated three times each with 25 ml of ethanol, the solvent being reevaporated after each treatment. The residue was then dissolved in 12 ml of water and the pH adjusted to 6-7 with saturated aqueous sodium bicarbonate. The precipitate was filtered off, washed with 2 ml of water and dried in vacuo to yield 1.60 g. Recrystallization from ethanol gave a pure product, m.p. 161-3°C (a sample for analysis had m.p. 162-3°C).

Analyses - Found: C 46.96; H 5.35; N 24.77; O 22.60%.

Calculated for C₁₁H₁₅N₅O₄: C 46.97; H 5.38; N 24.90; 0 22.75%.

### e) 9-(3,4-Dihydroxybutyl)guanine

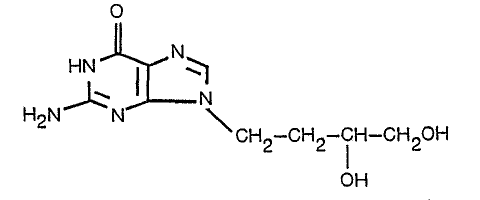

To a suspension of ethyl 4-(2-amino-1,6-dihydro-6-oxopurin-9-yl)-2-hydroxybutyrate (prepared according to d)) in isopropanol was added an excess of sodium borohydride and the mixture was refluxed over night (at least 8 hours). Hydrochloric acid was added until a clear solution was obtained (neutral pH). After removal of the solvent the residue was dissolved in a minimum amount of boiling water and kept at 0°C for a couple of hours. The solid was filtered off. The filtrate was evaporated at reduced pressure and the residue dissolved in hydrochloric acid (1 mol/litre) and adsorbed on a cation exchange resin (Dowex 50 W, H⁺⁻form).

The resin was washed with water and then eluted with 5% ammonium hydroxide. The eluent was evaporated to give a crystalline solid which was recrystallized from water to afford colourless needles. M.p. 260―1°C (dec.) (uncorrected) UV spectrum (hydrochloric acid 0.01 mol/litre): λₘₐₓ (nm) 277,253 (s = 11500) M.S.: 11.2 a J. (int): 239 (M⁺, 0.13), 222 (0.19), 221 (0.11), 152 (0.43), 151 (0.56), 44 (1.0).

### f) 9-(4-t-Butyldimethylsilyloxy-3-hydroxybutyl) guanine

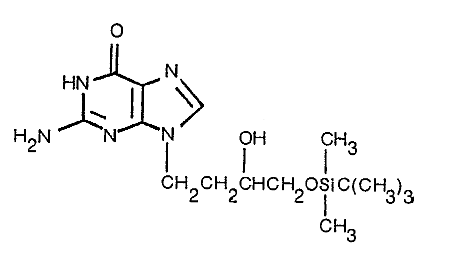

A mixture of 9-(3,4-dihydroxybutyl)guanine (66 mg, 0.276 mmol; prepared according to e)), t-butyldimethylchlorsilane (80 mg, 0.53 mmol), and imidazole (55 mg, 0.81 mmol) in 3.5 ml of dry dimethylformamide was stirred at room temperature for 2 h, and the solvent was evaporated in vacuo. The semi-solid residue was triturated with a little diethyl ether and aqueous sodium hydrogencarbonate solution, washed with water and dried in vacuo to yield 89 mg of a crude product. Chromatography (15 g of silica gel, chloroform-methanol 5:1 by volume) afforded 68 mg of mono-TBDMSi derivative. M.p. 235.5―240.5°C.

¹H NMR (DMSO-d₆): 6 0.02 (s, 6H), Si(CH₃)₂; 0.85 (s, 9H), C(CH₃)₃; 1.55―1.8 and 1.9―2.1 (2m, 2H), N-C-CH₂; 3.35-3.6 (m, 3H), CHCH₂; 4.0-4.1 (m, 2H), NCH₂; 6.3 (broad s, 2H), NH₂; 7.6 (s, 1H), H₈.

¹³C NMR (DMSO-d₆): The ¹³C NMR spectrum was in agreement with the postulated structure.

A downfield shift of C-4' from 65.925 [in 9-(3,4-dihydroxybutyl)guanine] to 67.336 ppm was observed.

### g) 9-(3-Thioacetyl 4-t-butyldimethylsilyloxybutyl)guanine

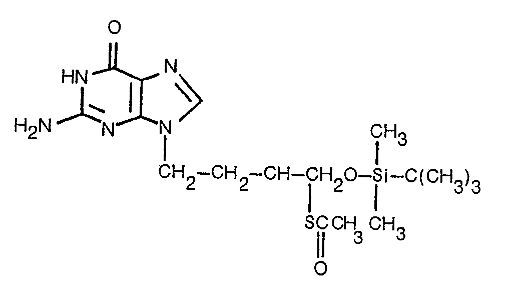

Diisopropylazodicarboxylate (75 mg, 0.37 mmol; cf. R. P. Volante, Tetrahedron letters vol. 22, No. 33, pp. 3119-3122, 1981) was added to a solution of triphenylphosphine (97.6 mg, 0.37 mmol) in dimethylformamide (2 ml) at 0°C. The mixture was stirred at 0°C for 30 min, 9-(3-hydroxy-4-t-butyldimethylsilyloxybutyl)guanine (50 mg, 0.16 mmol; prepared according to f)) in dimethylformamide (2 ml) and thioacetic acid in 1 ml dimethylformamide (28 mg, 0.37 mmol) was added dropwise and the solution was stirred at 0°C for 1 h, at 20°C over night and at 80°C for 4 hours. The solution was concentrated and purified by preparative thin layer chromatography (elution with chloroform-methanol 5:1) to yield 10 mg of 9-(3-thioacetyl-4-t-butyldimethylsilyloxybutyl)guanine. R_{f} 0.38 (chloroform-methanol 8:1).

¹H NMR (DMSO-d₆): δ 0.02 (s, 6H) (CH₃)₂Si; 0.85 (s, 9H) C(CH₃)₃; 2.3 (s, 3H) CH₃COS; 4.0 (AB quartet, 2H) NCH₂, 6.4 (s, 2H) H₂N; 7.7 (s, 2H) H₈.

### h) 9-(3-Mercapto-4-t-butyldimethylsilyloxybutyl)guanine

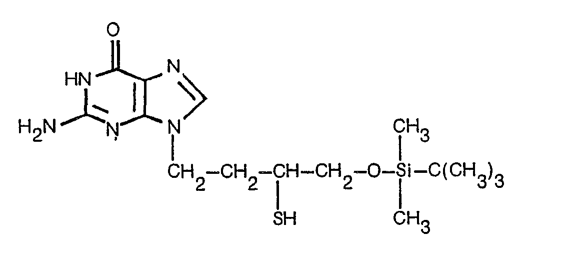

9-(3-Thioacetyl-4-t-butyldimethylsilyloxybutyl)guanine (10 mg; prepared according to g)) was dissolved in methanol (10 ml), saturated with ammonia and kept at room temperature for 1 h. Thin layer chromatography (chloroform-methanol 5:1) showed the absence of starting material and the solution was concentrated to give 9-(3-mercapto-4-t-butyldimethylsilyloxybutyl)guanine. R_{f} = 0.32 (chloroform-methanol 5:1). Mass spectrometry revealed the presence of a sulfhydryl group.

The following examples illustrate the preparation of pharmaceutical compositions of the invention. The wording "active substance" denotes a compound according to the present invention or a salt thereof.

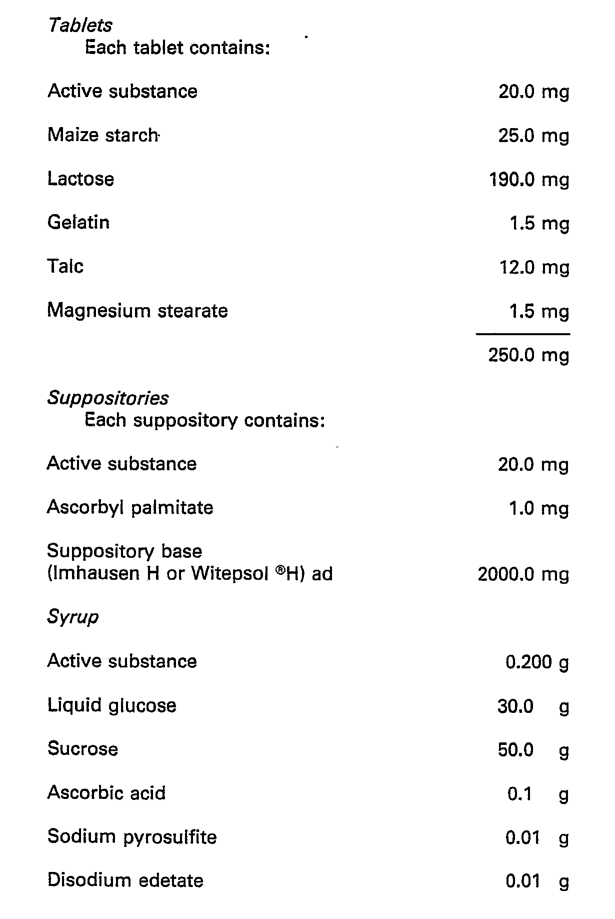

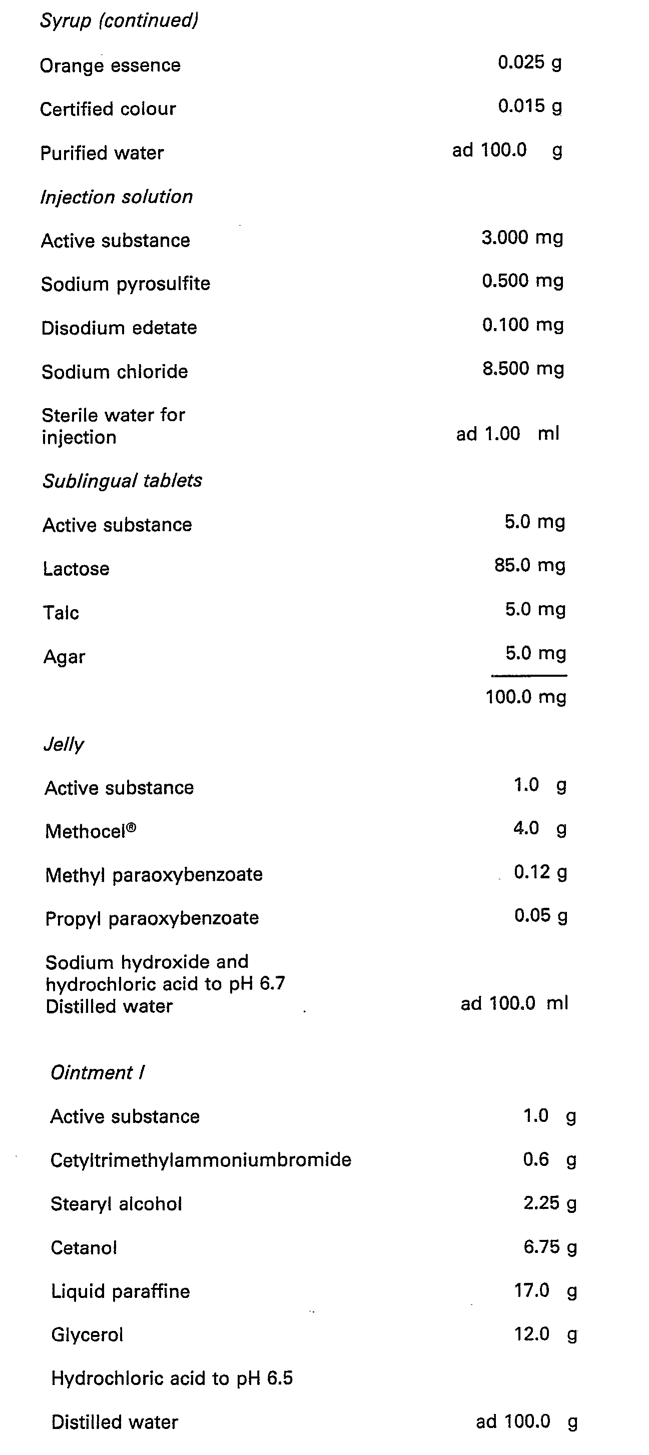

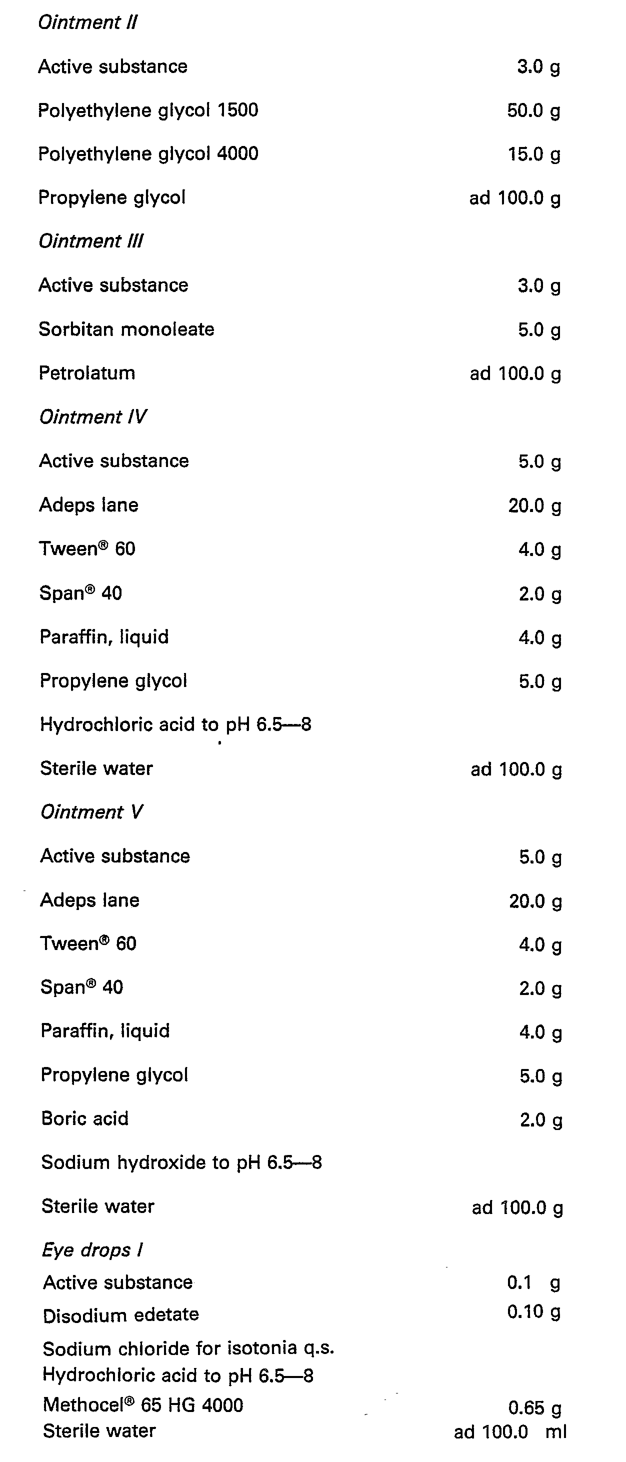

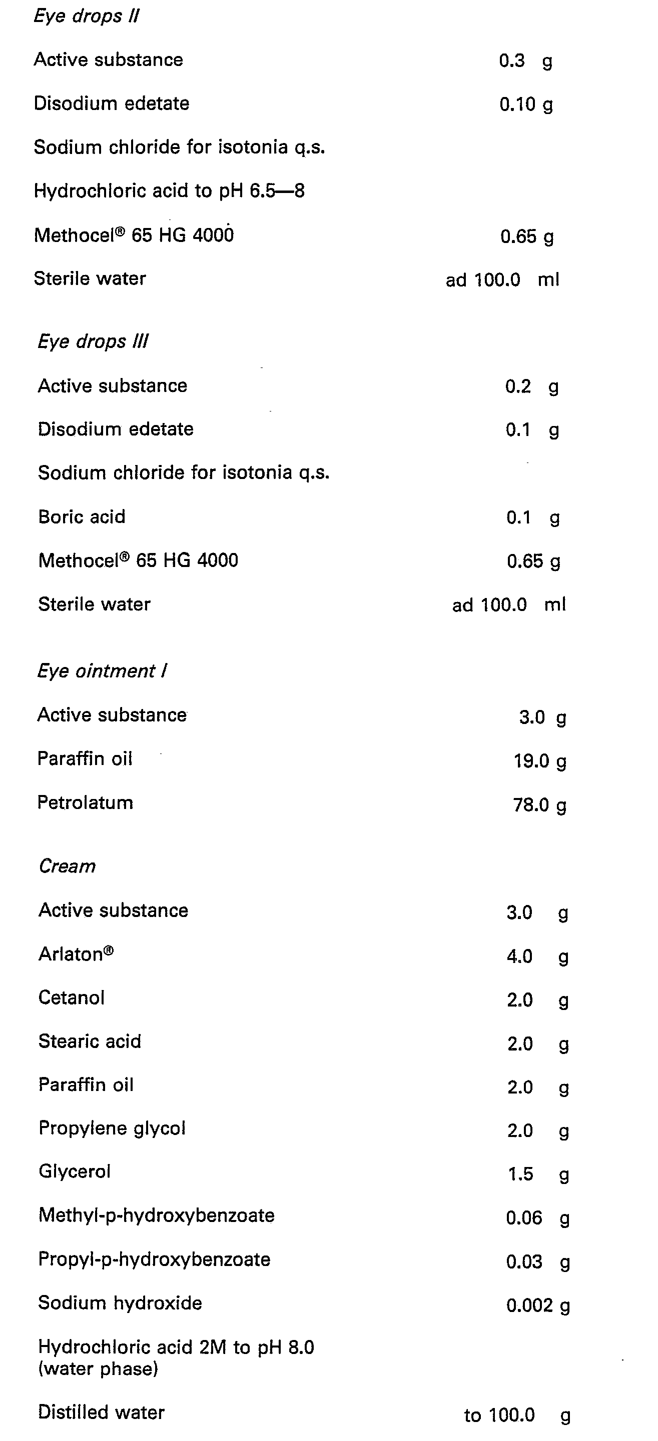

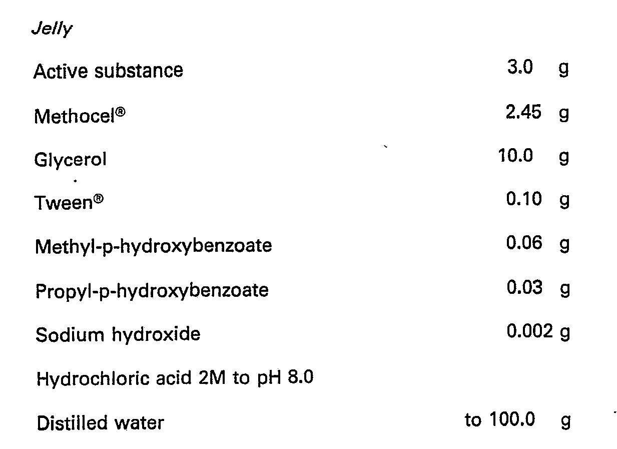

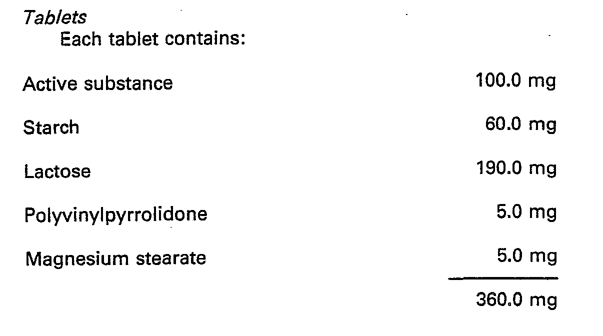

### Example 3

### ac. threo-9-(3,4-Dihydroxy-2-fluorobutyl)guanine

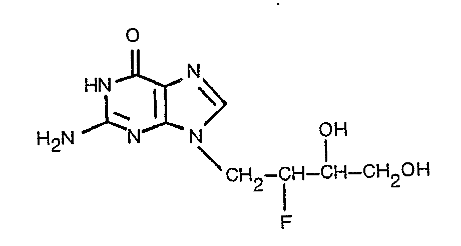

A solution of rac. threo-4-(2-amino-6-chloropurin-9-yl)-3-fluoro-O,O-isopropylidenebutane-1,2-diol (83 mg) in 1 ml of 2 M hydrochloric acid was kept at 100°C for 2 h and then evaporated to dryness in vacuum. The residue was dissolved in 10 ml of water and the solution neutralized by addition of weak anion exchange resin (OH form), heated to boiling and filtered warm. Upon cooling 42 mg (62%) of white crystals were obtained. Mp. ca. 277°C.

UV spectrum, lambda-max (nm): 0.01 M HCl, 253 (276); H₂O (pH 7), 251 (270 infl.); 0.01 M NaOH, 266 (255 infl.).

¹³C NMR, 50.10 MHz (DMSO-d₆): ppm 157.02 (C6); 153.84 (C2); 151.57 (C4); 137.93 (C8); 116.69 (C5); 92.49, 88.94 (J_{C-F} 178 Hz, CHF); 70.56, 70.18 (J_{C-C-F} 18.3 Hz, CHO); 61.52, 61.42 (J_{C-C-C-F} 4.9 Hz, CH₂O); 44.23, 43.76 (J_{C-C-F} 23.2 Hz, NCH₂).

The starting compound rac. threo-4-(2-amino-6-chloropurin-9-yl)-3-fluoro-0,0-isopropylidenebutane-1,2-diol was prepared as follows.

### a) rac 1.2-Dideoxy-1-bromo-2-fluoro-3,4-O-isopropylidenethreitol

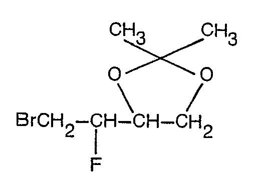

This compound was synthesized in 45% yield from rac. 2-deoxy-2-fluoro-3,4-0-isopropylidenethreitol (R. Cherry and P. W. Kent, J. Chem. Soc. 1962, 2507-09) by the method of R. Barner et al., Helv. Chim. Acta 64,926(1981).

¹³C NMR (CDCl₃): ppm 110.46 (C(CH₃)₂); 92.90, 89.25 (J_{C-F} 183 Hz, CHF); 75.26, 74.87 (J_{c-c-F} 19.5 Hz, CHO); 65.12, 65.00 (J_{C-C-C-F} 6.1 Hz, CH₂O); 29.58, 29.05 (J_{C-C-F} 27 Hz, CH₂Br); 26.17, 25.47 (C(CH₃)₂).

### b) rac. threo-4-(2-Amino-6-chloropurin-9-yl)-3-fluoro-O,O-isopropylidenebutane-1,2-diol

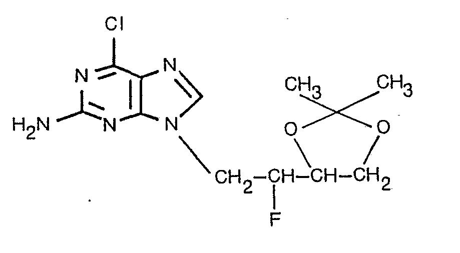

A mixture of rac. 1,2-dideoxy-1-bromo-2-fluoro-3,4-O-isopropylidenethreitol (325 mg, 1.43 mmol), 2-amino-6-chloropurine (300 mg, 1.77 mmol), finely ground, anhydrous potassium carbonate (244 mg, 1.77 mmol), and sodium iodide (120 mg) in 5 ml of dry dimethylformamide was stirred at room temperature for 70 h. After addition of water (10 ml) the mixture was washed with 3 x 10 ml of n-hexane and then extracted with 3 x 10 ml of dichloromethane. The CH₂CI₂ extracts were dried (MgS0₄) and evaporated in vacuum. Flash chromatography (silica gel, chloroform-methanol 15:1) afforded 164 mg (44%) of rac. threo-4-(2-amino-6-chloropurin-9-yl)-3-fluoro-0,0-isopropylidenebutane-1,2-diol.

¹³C NMR (CDCl₃): ppm 159.40 (C2); 153.81, 151.21 (C6―C4); 142.76 (C8); 110.36 (C(CH₃)₂); 90.81,87.18 (J_{C-F} 182 Hz, CHF); 74.51, 74.12 (J_{C-C-F} 19.5 Hz, CHO); 64.71, 64.61 (J_{C-C-C-F} 4.9 Hz, CH₂O); 44.64,44.18 (J_{C-C-F} 23 Hz, NCH₂); 25.91, 25.18 (C(CH₃)₂).

### Example 4

### rac. erythro-9-(3,4-Dihydroxy-2-methoxybutyl)guanine

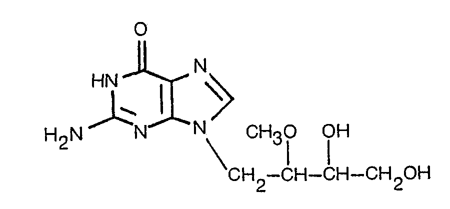

A solution of rac. erythro-4-(2-amino-6-chloropurin-9-yl)-3-methoxy-0,0-isopropylidenebutane-1,2-diol (15 mg) in 1 ml of 1 M hydrochloric acid was kept at 100°C for 1 h and then evaporated in vacuum, reevaporated with 3 x 1 ml of water and the residue dissolved in 1 ml of water, neutralized with aqueous ammonia and cooled to yield 11 mg of white crystals.

¹³C NMR, 50.10 MHz (DMSO-d₆): ppm 156.90 (C6); 153.79 (C2); 151.55 (C4); 138.22 (C8); 116.40 (C5); 80.01 (CH-OCH3); 71.45 (CHOH); 62.76 (CH₂OH); 57.90 (OCH₃); 43.13 (NCH₂).

The starting compound rac. erythro-4-(2-amino-6-chloropurin-9-yl)-3-methoxy-0,0-isopropylidenebutane-1,2-diol was prepared as follows:

### a) rac. 2-O-methyl-1,3 4-tribenzoylerythritol

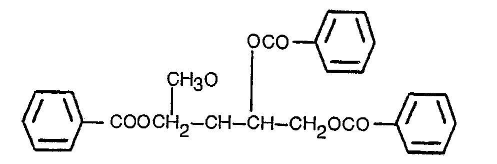

rac. 1,2,4-O-Tribenzoylerythritol (690 mg, 1.59 mmol), methyl iodide (0.30 ml, 4.76 mmol), and silver oxide (737 mg, 3.18 mmol) were stirred in 16 ml of dimethylformamide at room temperature for 24 h. The mixture was filtered through Celite and evaporated in vacuum. Flash chromatography (silica gel, n-hexane- ethyl acetate 4:1) afforded 230 mg of pure rac. 2-0-methyl-1,3,4-tribenzoylerythritol.

¹³C NMR (CDCl₃): ppm 166.09, 166.02, 165.41 (carbonyls); 133.18, 133.01, 129.65, 129.58, 128.34 (phenyl); 78.16 (CH-OCH₃); 71.13 (CH-OCOC₆H₅); 63.10, 62.59 (2 CH₂); 58.77 (OCH₃).

### b) rac. 2-O-Methylerythritol

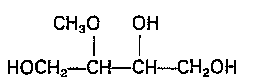

rac. 2-O-Methyl-1,3,4-tribenzoylerythritol (230 mg, 0.51 mmol) and sodium methoxide (15 mg) were stirred in.5 ml of anhydrous methanol at room temperature for 70 min. and then evaporated to dryness. The residue was dissolved in water, washed twice with carbon tetrachloride and evaporated to yield 69 mg of product.

¹³C NMR (CD₃OD): ppm 83.79 (CH―OCH3); 72.82 (CHOH); 64.55, 61.63 (2 CH₂); 58.67 (OCH₃).

### c) rac. 2-0-Methyl-3,4-0-isopropylidenerythritol

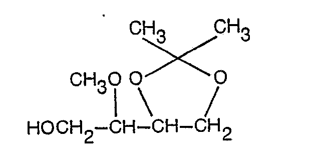

A solution of rac. 2-0-methylerythritol (69 mg, 0.39 mmol) and 3 drops of 70% perchloric acid in 5 ml of acetone was stirred at room temperature for 2.5 h, neutralized with saturated aqueous sodium bicarbonate and evaporated, dissolved in ethyl acetate, washed with a small volume of brine, dried (Na₂SO₄), and evaporated to afford the isopropylidene derivative in quantitative yield.

¹³C NMR (CDCl₃): ppm 109.24 (C(CH₃)₂); 82.05 (CH―OCH3); 75.70 (CHO); 66.89 (CH₂O); 61.20 (CH₂OH); 58.38 (OCH₃); 26.61, 25.20 (C(CH₃)₂).

### d) rac. 1-Deoxy-1-bromo-2-O-methyl 3,4-O-isopropylidenerythritol

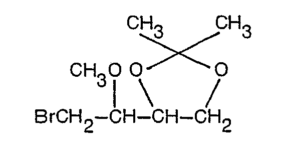

This compound was synthesized in 39% yield from rac. 2-0-methyl-3,4-0-isopropylidenerythritol as described for example 3 a).

¹³C NMR (CDCl₃): ppm 109.66 (C(CH₃)₂); 80.64 (CH-OCH₃); 75.75 (CHO); 66.87 (CH₂O); 58.09 (OCH₃); 32.28 (CH₂Br); 26.90, 25.27 (C(CH₃)₂).

### e) rac. erythro-4-(2 Amino-6-chloropurin-9-yl)-3-methoxy-0,0-isopropylidenebutane-1,2-diol

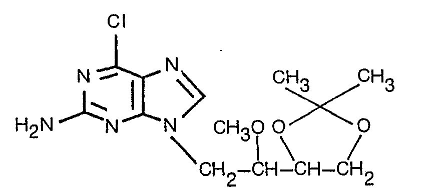

This compound was synthesized in 34% yield essentially as described for example 3b) with reaction temperature 90°C for 5 h.

¹³C NMR (CDCl₃): ppm 159.28 (C2); 143.62 (C8); 110.00 (C(CH₃)₂); 80.83 (CH―OCH₃); 75.21 (CHO); 66.90 (CH₂O); 58.97 (OCH₃); 43.79 (NCH₂); 26.76, 25.13 (C(CH₃)₂).

## Claims

1. A compound of the formula 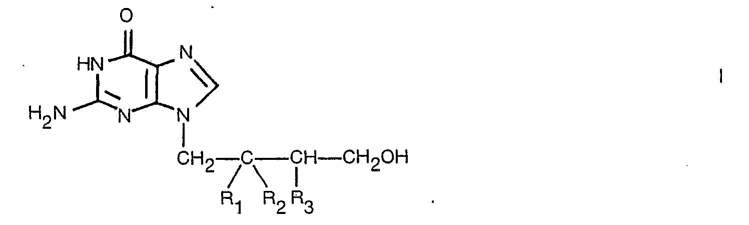 wherein R₁ is hydrogen, R₂ is hydrogen, fluoro, methoxy or methylthio and R₃ is hydrogen, hydroxy or mercapto; with the provisos that when R₃ is hydrogen, then R₂ is methoxy or methylthio and that when R₃ is hydroxy, then R₂ is fluoro, methoxy or methylthio; and with the further proviso that R₁ can also be methoxy or fluoro when R₂ is methoxy or fluoro; and physiologically acceptable salts or optical isomers thereof.

2. A compound according to claim 1 wherein in the formula I R₃ is hydroxy; and physiologically acceptable salts or optical isomers thereof.

3. A compound according to claim 1 wherein in the formula I R, is hydrogen, R₂ is hydrogen and R₃ is mercapto; and physiologically acceptable salts or optical isomers thereof.

4. A compound according to claim 1 wherein in the formula I R₁ is hydrogen, R₂ is fluoro and R₃ is hydroxy; and physiologically acceptable salts or optical isomers thereof.

5. A compound according to claim 1 wherein in the formula I R₁ is hydrogen, R₂ is methoxy and R₃ is hydroxy; and physiologically acceptable salts or optical isomers thereof.

6. A compound according to claim 1 wherein in the formula I R₁ and R₂ are methoxy and R₃ is hydrogen; and physiologically acceptable salts or optical isomers thereof.

7. A compound according to claim 1 wherein in the formula I R₁ and R₂ are methoxy and R₃ is hydroxy; and physiologically acceptable salts or optical isomers thereof.

8. A pharmaceutical composition comprising as an active ingredient a compound of the formula 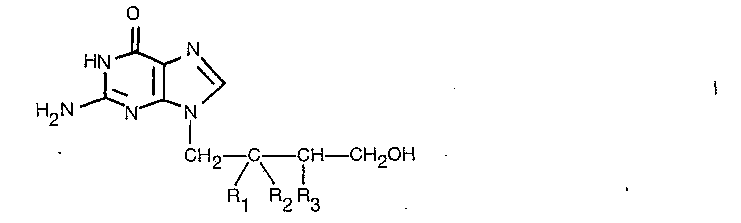 wherein R, is hydrogen, R₂ is hydrogen, fluoro, methoxy or methylthio and R₃ is hydrogen, hydroxy or mercapto; with the provisos that when R₃ is hydrogen then R₂ is methoxy or methylthio and that when R₃ is hydroxy then R₂ is fluoro, methoxy or methylthio; and with the further proviso that R, can also be methoxy or fluoro when R₂ is methoxy or fluoro; or a physiologically acceptable salt or an optical isomer thereof; in conjunction with a pharmaceutically acceptable carrier.

9. A pharmaceutical composition according to claim 8 designed for systemic administration.

10. A pharmaceutical composition according to claim 8 designed for topical administration.

11. A compound according to any of claims 1―7 for use in the treatment of virus infections.

12. A compound according to claim 11 for use in the treatment of herpes virus infections.

13. A compound according to any of claims 1-7 for use in the treatment of neoplastic diseases.

14. A method for the preparation of a compound of the formula 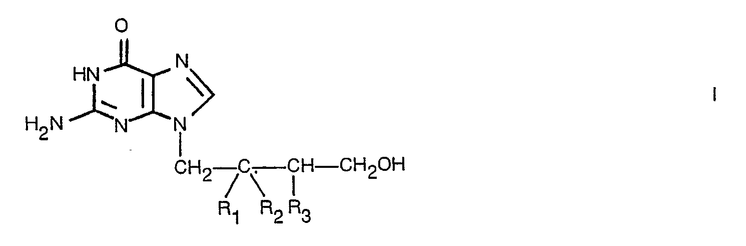 wherein R, is hydrogen, R₂ is hydrogen, fluoro, methoxy or methylthio and R₃ is hydrogen, hydroxy or mercapto; with the proviso that when R₃ is hydrogen then R₂ is methoxy or methylthio and that when R₃ is hydroxy then R₂ is fluoro, methoxy or methylthio; and with the further proviso that R, can also be methoxy or fluoro when R₂ is methoxy or fluoro; or a physiologically acceptable salt or an optical isomer, comprising
A. Condensing an acyclic side chain of the formula 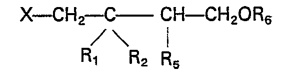 to the N-9 position of a guanine derivative of the formula: 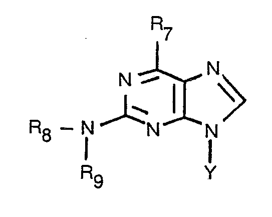 followed by removal of possible protecting groups, in which formulas R₁ and R₂ have the meanings given above, X is chlorine, bromine, iodine or a group OSO₂R₁₀ where R₁₀ is alkyl containing 1―8 carbon atoms, fluorinated alkyl containing 1-8 carbon atoms, alkylaryl or aryl; Y is hydrogen or a quarternary ammonium ion; R₅ is R₃ as defined above, or OR₆ or SR₆ where R₆ is hydrogen or a hydroxyl or a mercapto protecting group, R₅ and OR₆ may optionally together form an epoxide when R₃ is OH, or R₅ and OR₆ may optionally form a cyclic derivative such as a carbonate ester, carbonate thioester or the corresponding orthoacid cyclic derivatives or cyclic acetal type compounds, R₇ is hydroxyl, chlorine, bromine, iodine, thiol, thioether, SO₂R₁₀ where R₁₀ is as defined above; or an oxygen derivative OR₁₂ where R₁₂ is alkyl, alkylaryl, substituted silyl, phosphoryl diester, phosphinothioyl or SO₂R₁₀ where R₁₀is as defined above; R₈ and R₉ are the same or different and are R¹¹ where R¹¹ is hydrogen or an amine protecting group; or
B1. Substitution in the guanine N-9 derivatized 4-carbon side-chain of a compound of the formula 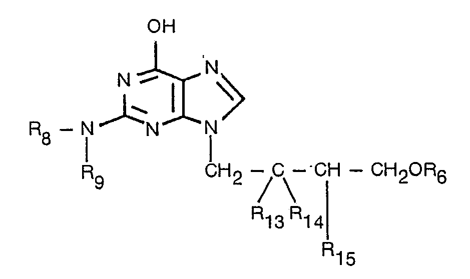 to form a compound of the formula 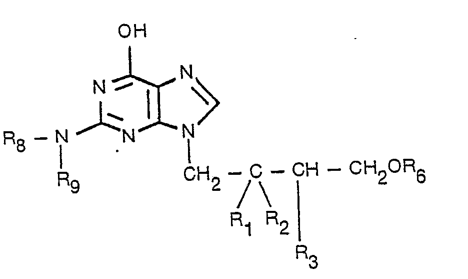 followed by removal of possible protecting groups, in which formula R₆, R₈ and R₉ are as defined above; R₁₃ is R₁, iodine or OSO₂R₁₀; R₁₄ is R₂, iodine or OSO₂R₁₀; R₁₅ is R₃, iodine or OSO₂R₁₀; and R₁, R₂, R₃ and R₁₀ are as defined above; or
B2. Reducing the ester group to an alcohol in a compound of the formula 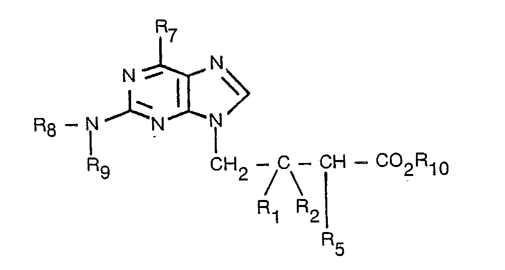 followed by removal of possible protecting groups, in which formula R₁, R₂, R₅, R₇, R₈, R₉ and R₁₀ are as defined above; or
C1. Ring closure of a compound of the formula 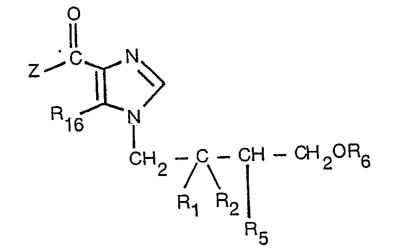 followed by removal of possible protecting groups, in which formula R₁, R₂, R₅ and R₆ are as defined above, Z is NH₂ or an alkoxy group, and R₁₆ is NH₂ or guanidino; or
C2. Ring closure of a compound of the formula 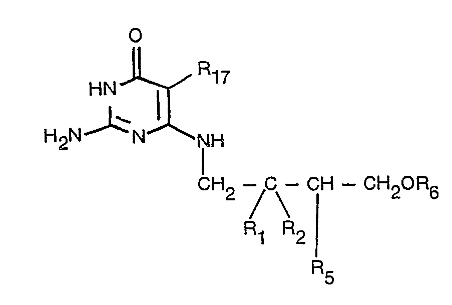 followed by removal of possible protecting groups, in which formula R₁, R₂, R₅ and R₆ are as defined above, and R₁₇ is nitroso, nitro, amino, formic amide or amino ortho ester; or
D. substitution in the pyrimidine ring of a compound of the formula 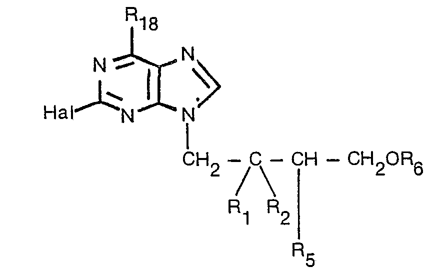 to form a compound of formula: 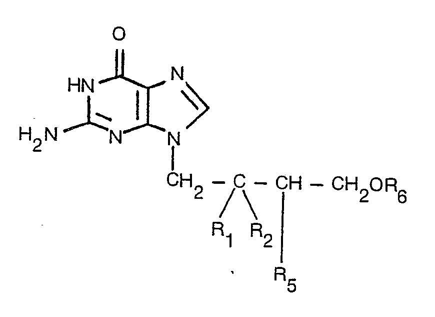 followed by removal of possible protecting groups, in which formula R₁, R₂, Rₛ and R₆ have the meaning given above, Hal is fluoro, chloro, bromo or iodine, R₁₈ is hydroxyl or amino, whereby when R₁₈ is amino the amino group is converted to a hydroxyl function;
to form a compound of the formula I, whereupon optionally a base obtained is converted to a pharmaceutically acceptable salt or a salt obtained is converted to the base or to a different, pharmaceutically acceptable salt, and optionally an isomeric mixture obtained is separated into a pure enantiomeric isomer.

15. A method according to claim 14 for the preparation of a compound of any of claims 2-7.

16. A compound according to any of claims 1-7 for use as a therapeutic agent.

17. A compound according to any of claims 1―7 for use as a prophylactic agent in the medical field.

## Patentansprüche

1. Verbindung der Formel 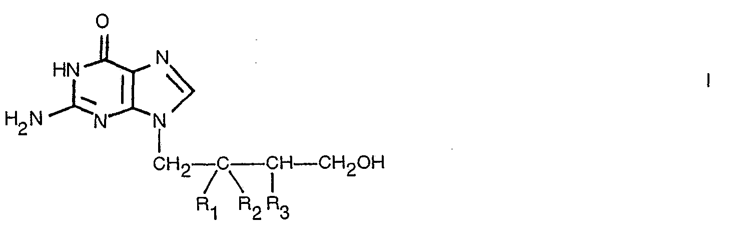 worin R₁ Wasserstoff, R₂ Wasserstoff, Fluor, Methoxy oder Methylthio·und R₃ Wasserstoff, Hydroxy oder Mercapto bedeuten, mit den Maßgaben, daß R₂ für Methoxy oder Methylthio steht, wenn R₃ Wasserstoff bedeutet, und R₂ für Fluor, Methoxy oder Methylthio steht, wenn R₃ Hydroxy bedeutet; und mit der weiteren Maßgabe, daß R₁ auch Methoxy oder Fluor sein kann, wenn R₂ für Methoxy oder Fluor steht; und physiologisch akzeptable Salze oder optische Isomeren derselben.

2. Verbindung nach Anspruch 1, worin R₃ in der Formel I für Hydroxy steht; und physiologisch akzeptable Salze oder optische Isomeren derselben.

3. Verbindung nach Anspruch 1, worin in der Formel I R₁ für Wasserstoff, R₂ für Wasserstoff und R₃ für Mercapto stehen; und physiologisch akzeptable Salze oder optische Isomeren derselben.

4. Verbindung nach Anspruch 1, worin in der Formel I R₁ für Wasserstoff, R₂ für Fluor und R₃ für Hydroxy stehen; und physiologisch akzeptable Salze oder optische Isomeren derselben.

5. Verbindung nach Anspruch 1, worin in der Formel I R₁ für Wasserstoff, R₂ für Methoxy und R₃ für Hydroxy stehen; und physiologisch akzeptable Salze oder optische Isomeren derselben.

6. Verbindung nach Anspruch 1, worin in der Formel I R₁ und R₂ Methoxy bedeuten und R₃ für Wasserstoff steht; und physiologisch akzeptable Salze oder optische Isomeren derselben.

7. Verbindung nach Anspruch 1, worin in der Formel I R₁ und R₂ Methoxy bedeuten und R₃ für Hydroxy steht; und physiologisch akzeptable Salze oder optische Isomeren derselben.

8. Pharmazeutische Zusammensetzung, welche als aktives Ingredienz eine Verbindung der Formel 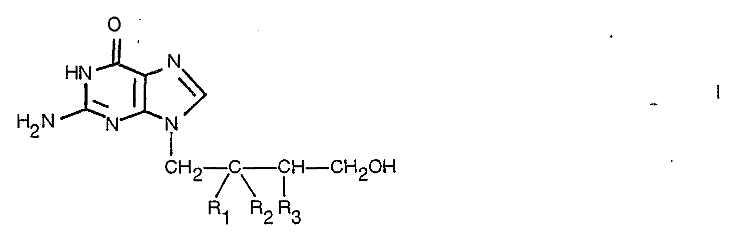 worin R₁ Wasserstoff, R₂ Wasserstoff, Fluor, Methoxy oder Methylthio und R₃ Wasserstoff, Hydroxy oder Mercapto bedeuten, mit den Maßgaben, daß R₂ für Methoxy oder Methylthio steht, wenn R₃ Wasserstoff bedeutet, und R₂ für Fluor, Methoxy oder Methylthio steht, wenn R₃ Hydroxy bedeutet; und mit der weiteren Maßgabe, daß R₁ auch Methoxy oder Fluor sein kann, wenn R₂ für Methoxy oder Fluor steht; oder ein physiologisch akzeptables Salz oder ein optisches Isomeres derselben in Verbindung mit einem pharmazeutisch akzeptablen Träger enthalt.

9. Pharmazeutische Zusammensetzung nach Anspruch 8 für systemische Verabreichung.

10. Pharmazeutische Zusammensetzung nach Anspruch 8 für topische Verabreichung.

11. Verbindung nach einem der Ansprüche 1 bis 7 zur Verwendung bei der Behandlung von Virusinfektionen.

12. Verbindung nach Anspruch 11 zur Verwendung bei der Behandlung von Herpes-Virusinfektionen.

13. Verbindung nach einem der Ansprüche 1 bis 7 zur Verwendung. bei der Behandlung von neoplastischen Krankheiten.

14. Verfahren zur Herstellung einer Verbindung der Formel 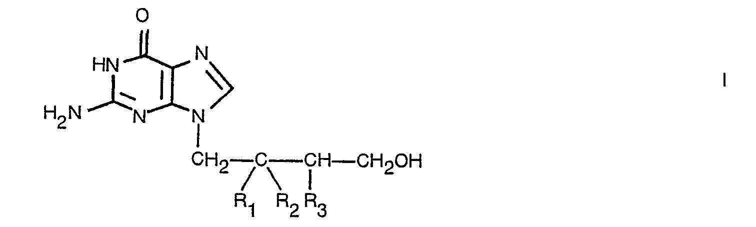 worin R₁ Wasserstoff, R₂ Wasserstoff, Fluor, Methoxy oder Methylthio und R₃ Wasserstoff, Hydroxy oder Mercapto bedeuten, mit der Maßgabe, daß R₂ für Methoxy oder Methylthio steht, wenn R₃ Wasserstoff bedeutet, und R₂ für Fluor, Methoxy oder Methylthio steht, wenn R₃ Hydroxy bedeutet; und mit der weiteren Maßgabe, daß R₁ auch Methoxy oder Fluor sein kann, wenn R₂ für Methoxy oder Fluor steht; oder eines physiologisch akzeptablen Salzes oder optischen Isomeren derselben, umfassend
A. Kondensieren einer azyklischen Seitenkette der Formel 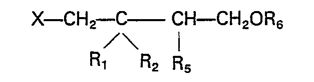 an die N-9 Position eines Guaninderivats der Formel 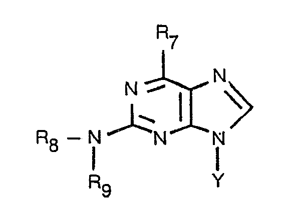 gefolgt vom Entfernen möglicher Schutzgruppen, in welchen Formeln R₁ und R₂ die oben angegebenen Bedeutungen haben, X für Chlor, Brom, Jod oder eine OSO₂R₁₀ Gruppe steht, wobei R₁₀ Alkyl mit 1―8 Kohlenstoffatomen, fluoriertes Alkyl mit 1-8 Kohlenstoffatomen, Alkylaryl oder Aryl bedeutet; Y für Wasserstoff oder ein quaternäres Ammoniumion steht; Rₛ für R₃ mit der obigen Bedeutung oder für OR₆ oder SR₆ steht, wobei R₆ Wasserstoff oder eine Hydroxyl- oder eine Mercaptoschutzgruppe repräsentiert; R₅ und OR₆ gewünschtenfalls gemeinsam ein Epoxid bilden können, wenn R₃ OH ist, oder R₅ und OR₆ gewünschtenfalls ein zyklisches Derivat, wie einen Carbonatester, Carbonatthioester oder die entsprechenden Orthosäure-zyklischen Derivate oder zyklische acetalartige Verbindungen bilden können; R₇ für Hydroxyl, Chlor, Brom, Jod, Thiol, Thioäther, SO₂R₁₀ steht, wobei R₁₀ wie oben definiert ist oder ein Sauerstoffderivat OR₁₂ ist, wobei R₁₂ Alkyl, Alkylaryl, substituiertes Silyl, Phosphoryldiester, phosphinothioyl oder SO₂R₁₀ ist, wobei R₁₀ die obige Bedeutung hat, R₈ und R₉ gleich oder verschieden sind und R₁₁ bedeuten, wobei R₁₁ Wasserstoff oder eine Aminschutzgruppe ist; oder
B1. Substitution an der N-9 derivatisierten 4-Kohlenstoffseitenkette des Guanins einer Verbindung der Formel 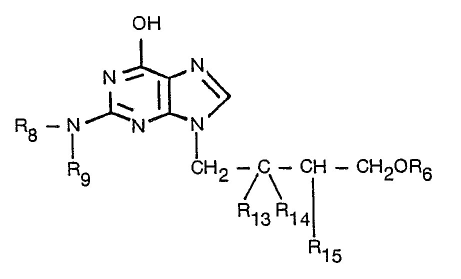 zur Bildung einer Verbindung der Formel 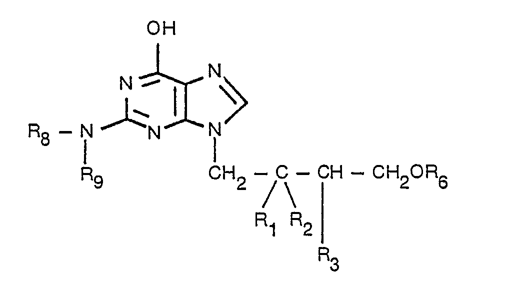 gefolgt vom Entfernen möglicher Schutzgruppen, in welcher Formel R₆, R₈ und R₉ wie oben definiert sind, R₁₃ für R₁, Jod oder OSO₂R₁₀ steht; R₁₄ R₂, Jod oder OSO₂R₁₀ bedeutet; R₁₅ für R₃, Jod oder OSO₂R₁₀ steht; und R₁, R₂, R₃ und R₁₀ wie oben definiert sind; oder
B2. Reduktion der Estergruppe zu einem Alkohol in einer Verbindung der Formel 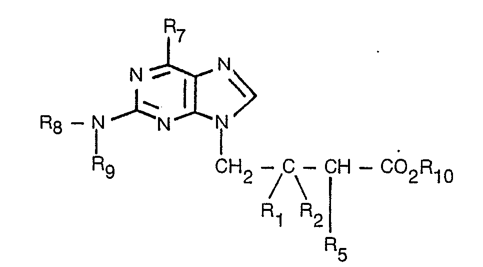 gefolgt vom Entfernen möglicher Schutzgruppen, in welcher Formel R₁, R₂, R₅, R₇, R₈, R₉ und R₁₀ wie oben definiert sind; oder
C1. Ringschluß einer Verbindung 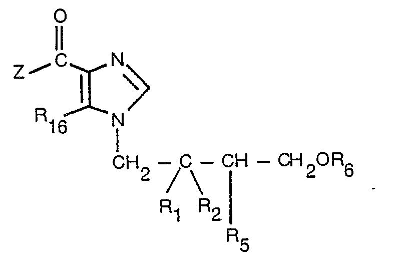 gefolgt vom Entfernen möglicher Schutzgruppen, in welcher Formel R₁, R₂, R₅ und R₆ die obige Bedeutung haben, Z für NH₂ oder eine Alkoxygruppe steht und R₁₆ NH₂ oder Guanidino repräsentiert; oder
C2. Ringschluß einer Verbindung der Formel 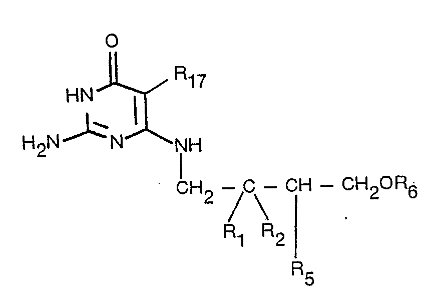 gefolgt vom Entfernen möglicher Schutzgruppen, in welcher Formel R₁, R₂, R₅ und R₆ die obige Bedeutung haben, und R₁₇, Nitroso, Nitro, Amino, Formamido oder Aminoorthoester repräsentiert; oder
D. Substitution im Pyrimidinring einer Verbindung der Formel 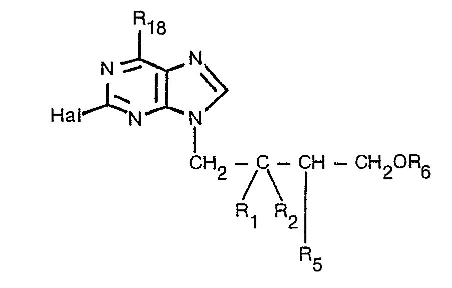 zur Bildung einer Verbindung der Formel 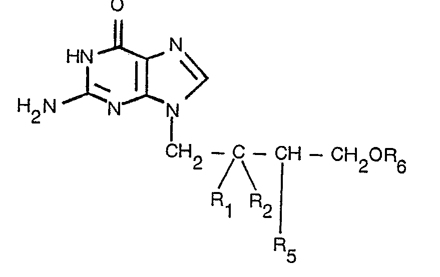 gefolgt vom Entfernen möglicher Schutzgruppen, in weicher Formel R₁, R₂, R₅ und R₆ die oben angegebene Bedeutung haben, Hai Fluor, Chlor, Brom oder Jod darstellt, R₁₈ für Hydroxyl oderAmino steht, wobei die Aminogruppe in eine Hydroxylfunktion übergeführt wird, wenn R₁₈ Amino repräsentiert;
zur Bildung einer Verbindung der Formel I, worauf gewünschtenfalls eine erhaltene Base in ein pharmazeutisch akzeptables Salz übergeführt wird oder ein erhaltenes Salz in die Base oder ein anderes pharmazeutisch akzeptables Salz übergeführt wird, und gewünschtenfalls eine erhaltene Isomerenmischung in ein reines enantiomeres Isomeres getrennt wird.

15. Verfahren nach Anspruch 14 zur Herstellung einer Verbindung nach irgendeinem der Ansprüche 2-7.

16. Verbindung nach irgendeinem der Ansprüche 1-7 zur Verwendung als ein therapeutisches Mittel.

17. Verbindung nach irgendeinem der Ansprüche 1-7 zur Verwendung als ein prophylaktisches Mittel für das medizinische Anwendungsgebiet.

## Revendications

1. Composé de formule 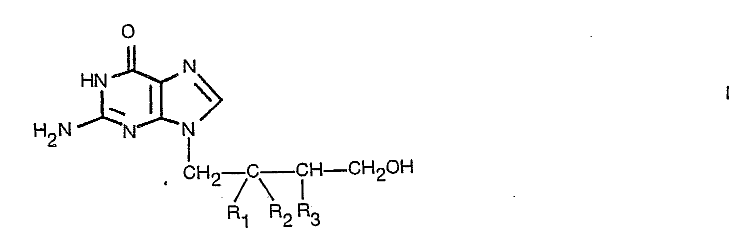 dans laquelle R₁ est l'hydrogène, R₂ est l'hydrogène, un groupe fluoro, méthoxy ou méthylthio et R₃ est l'hydrogène, un groupe hydroxy ou mercapto; à condition que, lorsque R₃ est l'hydrogène, R₂ soit alors un groupé méthoxy ou méthylthio et que, lorsque R₃ est un groupe hydroxy, R₂ soit alors un groupe fluoro, méthoxy ou méthylthio; et à condition en outre que R, puisse aussi être un groupe méthoxy ou fluoro lorsque R₂ est un groupe méthoxy ou fluoro; et sels physiologiquement acceptables ou isomères optiques de ce composé.

2. Composé selon la revendication 1, dans lequel, dans la formule I, R₃ est un groupe hydroxy; et sels physiologiquement acceptables ou isomères optiques de ce composé.

3. Composé selon la revendication 1, dans lequel, dans la formule I, R₁ est l'hydrogène, R₂ est l'hydrogène et R₃ est un groupe mercapto; et sels physiologiquement acceptables ou isomères optiques de ce composé.

4. Composé selon la revendication 1, dans lequel, dans la formule I, R₁ est l'hydrogène, R₂ est un groupe fluoro et R₃ est un groupe hydroxy; et sels physiologiquement acceptables ou isomères optiques de ce composé.

5. Composé selon la revendication 1, dans lequel, dans la formule I, R₁ est l'hydrogène, R₂ est un groupe méthoxy et R₃ est un groupe hydroxy; et sels physiologiquement acceptables ou isomères optiques de ce composé.

6. Composé selon la revendication 1, dans lequel, dans la formule 1, R₁ et R₂ sont des méthoxy et R₃ est l'hydrogène; et sels physiologiquement acceptables ou isomères optiques de ce composé.

7. Composé selon la revendication 1, dans lequel, dans la formule I, R₁ et R₂ sont un groupe méthoxy et R₃ est un groupe hydroxy; et sels physiologiquement acceptables ou isomères optiques de ce composé.

8. Composition pharmaceutique comprenant comme substance active un composé de formule 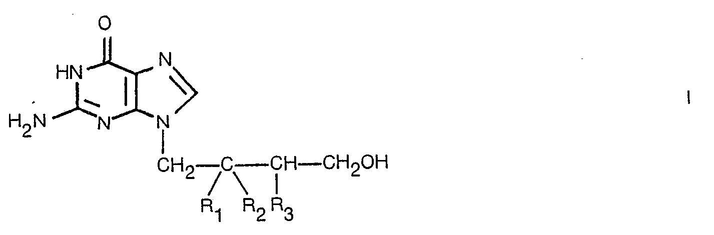 dans laquelle R₁ est l'hydrogène, R₂ est l'hydrogène, un groupe fluoro, méthoxy ou méthylthio et R₃ est l'hydrogène, un groupe hydroxy ou mercapto; à condition que, lorsque R₃ est l'hydrogène, R₂ soit alors un groupe méthoxy ou méthylthio et que, lorsque R₃ est un g'roupe hydroxy, R₂ soit alors un groupe fluoro, méthoxy ou méthylthio; et à condition en outre que R₁ puisse aussi être un groupe méthoxy ou fluoro lorsque R₂ est un groupe méthoxy ou fluoro; ou un sel physiologiquement acceptable ou un isomère optique de ce composé; en conjonction avec un véhicule pharmaceutiquement acceptable.

9. Composition pharmaceutique selon la revendication 8, conçue pour une administration générale.

10. Composition pharmaceutique selon la revendication 8, conçue pour une administration locale.

11. Composé selon l'une quelconque des revendications 1 à 7, utilisable dans le traitement des infections virales.

12. Composé selon la revendication 11, utilisable dans le traitement des infection virales de type herpès.

13. Composé selon l'une quelconque des revendications 1 à 7, utilisable dans le traitement des maladies néoplastiques.

14. Procédé de préparation d'un composé de formule 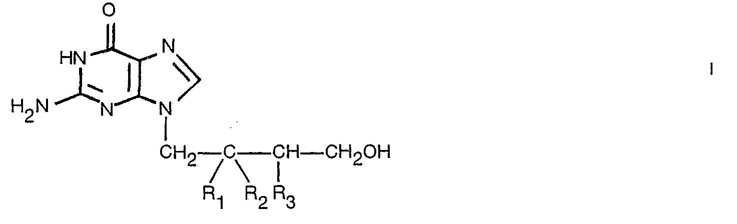 dans laquelle R₁ est l'hydrogène, R₂ est l'hydrogène, un groupe fluoro, méthoxy ou méthylthio et R₃ est l'hydrogène, un groupe hydroxy ou mercapto; à condition que, lorsque R₃ est l'hydrogène, R₂ soit alors un groupe méthoxy ou méthylthio et que, lorsque R₃ est un groupe hydroxy, R₂ soit alors un groupe fluoro, méthoxy ou méthylthio; et à condition en outre que R₁ puisse aussi être un groupe méthoxy ou fluoro lorsque R₂ est un groupe méthoxy ou fluoro; ou d'un sel physiologiquement acceptable ou d'un isomère optique de ce composé, comprenant
A. La condensation d'une chaîne latérale acyclique de formule 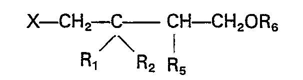 sur la position N-9 d'un dérivé de guanine de formule: 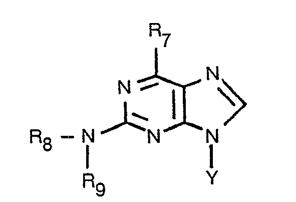 suivie de l'élimination des groupes protecteurs éventuels, formules dans lesquelles R₁ et R₂ ont les significations données ci-dessus, X est le chlore, le brome ou l'iode ou un groupe OSO₂R₁₀ dans lequel R₁₀ est un groupe alkyle contenant 1-8 atomes de carbone, alkyle fluoré contenant 1-8 atomes de carbone, alkylaryle ou aryle; Y est l'hydrogène ou un ion ammonium quaternaire; R₅ est R₃ tel que défini ci-dessus, ou OR₆ ou SR₆ dans lequel R₆ est l'hydrogène ou un groupe protecteur de fonction hydroxyle ou mercapto; R₅ et OR₆ peuvent éventuellement former ensemble un époxyde lorsque R₃ est OH, ou R₅ et OR₆ peuvent éventuellement former un dérivé cyclique comme un ester de carbone, un thioester de carbonate ou les dérivés orthoacides cycliques correspondants ou des composés de type acétal cyclique; R₇ est un groupe hydroxyle, chlore, brome, iode, thiol, thioéther, SO₂R₁₀ dans lequel R₁₀ est tel que défini ci-dessus; ou un dérivé oxygéné OR₁₂ dans lequel R₁₂ est un groupe alkyle, alkylaryle, silyle substitué, diester de phosphoryle, phosphinothioyle ou SO₂R₁₀ dans lequel R₁₀ est tel que défini ci-dessus; R₈ et R₉ sont identiques ou différents et sont R₁₁ dans lequel R₁₁ est l'hydrogène ou un groupe protecteur de fonction amine; ou
B1. Substitution dans la chaîne latérale à 4 carbones dérivée de la guanine en N-9 d'un composé de formule 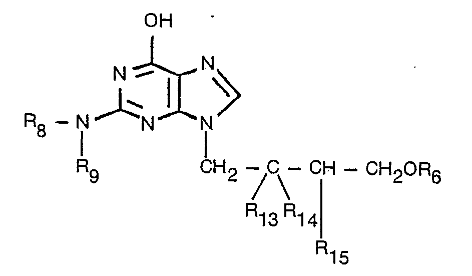 pour former un composé de formule 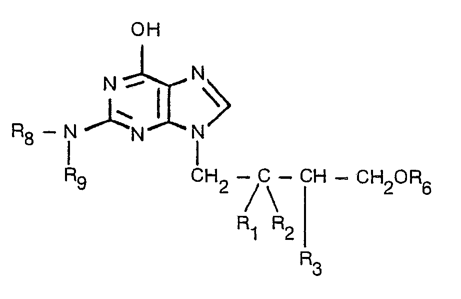 suivie de l'élimination des groupes protecteurs éventuels, formule dans laquelle R₆, R₈ et R₉ sont tels que définis ci-dessus; R₁₃ est R₁, l'iode ou OSO₂R₁₀; R₁₄ est R₂, l'iode ou OSO₂R₁₀; R₁₅ est R₃, l'iode ou OSO₂R₁₀; et R₁, R₂, R₃ et R₁₀ sont tels que définis ci-dessus; ou
B2. Réduction du groupe ester en alcool dans un composé de formule 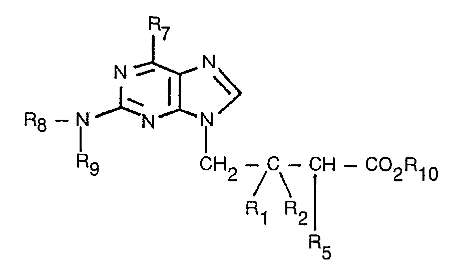 suivie de l'élimination des groupes protecteurs éventuels, formule dans laquelle R₁, R₂, R₅, R₇, R₈, R₉ et R₁₀ sont tels que définis ci-dessus; ou
C1. Fermeture de cycle d'un composé de formule 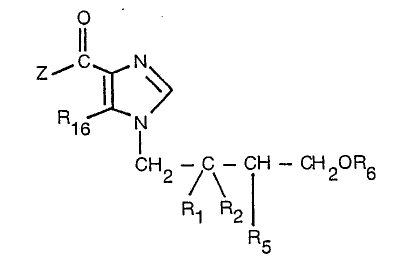 suivie de l'élimination des groupes protecteurs éventuels, formule dans laquelle R₁, R₂, R₅ et R₆ sont tels que définis ci-dessus, Z est NH₂ ou un groupe alcoxy et R₁₆ est NH₂ ou un groupe guanidino; ou
C2. Fermeture de cycle d'un composé de formule 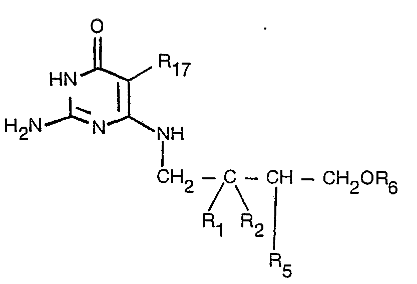 suivie de l'élimination des groupes protecteurs éventuels, formule dans laquelle R₁, R₂, R₅ et R₆ sont tels que définis ci-dessus et R₁₇ est un groupe nitroso, nitro, amino, amide formique ou amino-ortho-ester; ou
D. Substitution dans le cycle pyrimidine d'un composé de formule 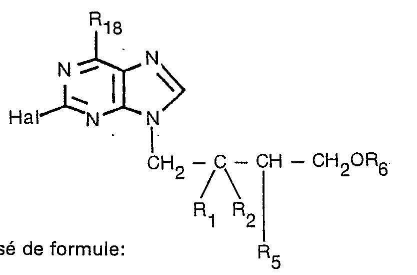 pour former un composé de formule: 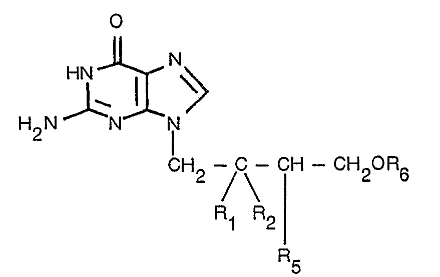 suivie de l'élimination des groupes protecteurs éventuels, formule dans laquelle R₁, R₂, R₅ et R₆ ont les significations données ci-dessus, Hal est un groupe fluoro, chloro, bromo ou iode, R₁₈ est un groupe hydroxyle ou amino, dans laquelle, lorsque R₁₈ est un groupe amino, le groupe amino est transformé en une fonction hydroxyle;
pour former un composé de formule I, la base obtenue étant éventuellement transformée en sel pharmaceutiquement acceptable ou le sel obtenu étant éventuellement transformé en base ou en un sel pharmaceutiquement acceptable différent et, éventuellement, le mélange isomère obtenu est séparé en énantiomère pur.

15. Procédé selon la revendication 14 pour la préparation d'un composé selon l'une quelconque des revendications 2 à 7.

16. Composé selon l'une quelconque des revendications 1 à 7, utilisable comme agent thérapeutique.

17. Composé selon l'une quelconque des revendications 1 à 7, utilisable comme agent prophylactique dans le domaine médical.
